# EUROPEAN PATENT SPECIFICATION

(11) **EP 1 301 786 B1**
(45) Date of publication and mention of the grant of the patent: **30.11.2005**
(21) Application number: 01969449.6
(22) Date of filing: 20.07.2001
(51) Int. Cl.: G01N 33/50, G01N 33/573, C07K 14/47

(54) **METHOD FOR THE DETECTION OF APOPTOSIS BY DETERMINING CYTOCHROME C AS APOPTOSIS-SPECIFIC MARKER RELEASED INTO AN EXTRACELLULAR MEDIUM THROUGH CELLULAR RELEASE MECHANISMS**
VERFAHREN ZUR DETEKTION DER APOPTOSE DURCH ERMITTLUNG VON CYTOCHROM C ALS APOPTOSESPEZIFISCHEM MARKER, DER DURCH ZELLULÄRE AUSSCHÜTTUNGSVORGÄNGE INS EXTRAZELLULÄRE MEDIUM ABGEGEBEN WIRD
METHODE DE DETECTION DE L'APOPTOSE PAR LA DETERMINATION DU CYTOCHROME C EN TANT QUE MARQUEUR SPECIFIQUE DE L'APOPTOSE LIBERE DANS UN MILIEU EXTRACELLULAIRE VIA DES MECANISMES DE LIBERATION CELLULAIRE

(30) Priority: 21.07.2000 EP 00115722; 12.09.2000 EP 00119818
(43) Date of publication of application: 16.04.2003
(73) Proprietor: Evotec AG, 22525 Hamburg (DE)
(72) Inventor: LOS, Marek, 48161 Münster (DE); RENZ, Andrea, 76135 Karlsruhe (DE); SCHULZE-OSTHOFF, Klaus, 48147 Münster (DE); BERDEL, Wolfgang, E., 48161 Münster (DE)
(74) Representative: Meyers, Hans-Wilhelm
(86) International application number: PCT/EP2001/008389
(87) International publication number: WO 2002/008752

(56) References cited:
- EP-A- 0 261 362
- WO-A-00/17648
- US-A- 3 642 978
- US-A- 4 617 187
- US-A- 5 928 860
- US-A- 5 972 622
- US-A- 6 048 703
- DATABASE BIOSIS [Online] BIOSCIENCES INFORMATION SERVICE, PHILADELPHIA, PA, US; September 2000 (2000-09) LOS M ET AL: "Cytochrome c is rapidly released from the cell upon apoptosis induction: A new marker for cell death in vivo." Database accession no. PREV200000468252 XP002184972 & IMMUNOLOGY LETTERS, vol. 73, no. 2-3, September 2000 (2000-09), page 239 24th European Immunology Meeting of the European Federation of Immunological Societies;Poznan, Poland; September 23-26, 2000 ISSN: 0165-2478
- DATABASE BIOSIS [Online] BIOSCIENCES INFORMATION SERVICE, PHILADELPHIA, PA, US; 1982 BLESCHKE H ET AL: "DIAGNOSIS AND THERAPY OF POISONING WITH GREEN BULBOUS AGARICS" Database accession no. PREV198274043349 XP002184973 & DEUTSCHE GESUNDHEITSWESEN, vol. 37, no. 5, 1982, pages 204-208, ISSN: 0012-0219

## Description

This invention relates to a method for the detection of apoptosis by determining apoptosis-specific markers. It further relates to a method for the identification of apoptosis-modulating substances and the use of an apoptosis detection kit for determining apoptosis-specific markers in a sample. Additionally, it relates to the use of cytochrome c and/or peptides derived thereof as a medicament or in pharmaceutical compositions.

Cell death occurs in two distinct forms in nature, namely necrosis and apoptosis. Necrosis results from massive physical or chemical insult, when noxious stimuli disintegrate the function of various cellular compartments leading to mitochondrial dysfunction, swelling of the cell followed by plasma membrane damage, and cell lysis. Necrosis generally provokes an inflammatory response. In contrast, apoptosis (programmed cell death) results from a gene-directed self-destruction program within the cells in response to internal and external stimuli. The apoptotic process is essential for development and maintenance of integrity of multicellular organisms. Through apoptosis cells produced in excess, that have been developed improperly, or with a genetic damage can be eliminated. Apoptotic cells (apoptotic bodies) are usually recognized and cleared by neighboring cells or macrophages without induction of an inflammatory response (Orrenius, S., 1995. Apoptosis: molecular mechanisms and implications for human disease. *J. Intern. Med.* 237:529-536). As virtually all cells in an organism can undergo apoptosis, the onset of the apoptotic process must be under permanent and tight physiological control.

Apoptotic cells are characterized by typical morphological and biochemical changes, like cytoskeletal disruption, cell shrinkage, membrane blebbing, DNA-fragmentation, and loss of phospholipid asymmetry with exposure of phosphatidyl serine (PS) on the outer leaflet of the cell membrane. Furthermore, during apoptosis cells maintain their membrane integrity (Thompson, C. B., 1995. Apoptosis in the pathogenesis and treatment of disease. *Science* 267:1456-1462).

Important key effector molecules in the apoptotic process are proteases called caspases (cysteinyl aspartate-specific proteinases). They contain a cysteine in the active center and cleave peptides and proteins at semiconserved sequences after specific aspartate residues. They are synthesized as zymogens which upon proteolytic activation form heterotetramers composed of two larger (p20) and two smaller (p10) subunits. During induction of apoptosis, they become (auto)activated and cleave a number of cellular proteins, thus participating in the occurrence of the typical morphology in apoptotic cells (Alnemri E.S., D.J. Livingston, D.W. Nicholson, G. Salvesen, N.A. Thornberry, W.W. Wong, and J. Yuan, 1996. Human ICE/CED-3 protease nomenclature. *Cell*, 87:171; Los, M., S. Wesselborg, and K. Schulze-Osthoff, 1999. The role of caspases in development, immunity, and apoptotic signal transduction: lessons from knockout mice. *Immunity* 10:629-639).

Induction of the endogenous apoptotic death machinery can be initiated via two principal distinct pathways (Budihardjo, I., H. Oliver, M. Lutter, X. Luo, and X. Wang, 1999. Biochemical pathways of caspase activation during apoptosis. *Annu. Rev. Cell Dev. Biol.* 15:269-290). One involves the ligation of death receptors, such as TRAIL-R1, TRAIL-R2, CD95, or TNF-R1, which upon binding of the adaptor protein FADD recruit procaspase-8 to the death-inducing signaling complex. Procaspase-8 is (auto)activated at the receptors and cleaves downstream targets, including other (effector) caspases. Another pathway that is triggered by a number of apoptotic stimuli, growth factor deprivation, excessive DNA-damage, anticancer drugs or irradiation, is essentially controlled by the mitochondrion. An initial event is the release of cytochrome c from mitochondria into the cytosol (reviewed in Bernhardi P., L. Scorrano, R. Colonna, V. Petronilli, and F. Di Lisa, 1999. Mitochondria and cell death. Mechanistic aspects and morphological issues. *Eur. J. Biochem.* 264: 687-701). Once released, cytochrome c together with (d)ATP binds to apoptotic protease-activating factor-1 (Apaf-1), leading to an unmasking of its caspase recruitment domain (CARD) followed by subsequent binding and autoproteolytic activation of procaspase-9. Procaspase-9, cytochrome c, (d)ATP, and Apaf-1 form the backbone of a protein complex, known as the apoptosome. Similarly to caspase-8, active caspase-9 then proteolytically activates downstream (effector) caspases, which by degrading various cellular proteins propagate the apoptotic signal. Both, the death receptor and the mitochondrial pathway can synergize and amplify their own signals by positive feedback loops. Firstly, the BH3-only Bax-interacting protein Bid, which is a substrate of caspase-8, becomes activated in the death receptor-mediated pathway and induces the release of cytochrome c from mitochondria (Li, H., H. Zhu, C.-J. Xu, and J. Yuan, 1998. Cleavage of BID by caspase 8 mediates the mitochondrial damage in the Fas pathway of apoptosis. *Cell* 94:491-501; Luo, X., I. Budihardjo, H. Zou, C. Slaughter, and X. Wang, 1998. Bid, a Bcl2 interacting protein, mediates cytochrome c release from mitochondria in response to activation of cell surface death receptors. *Cell* 94:481-490). Activated Bid triggers a conformational change of another pro-apoptotic molecule, Bax, which leads to its oligomerization, and subsequent insertion into the outer mitochondrial membrane and finally to cytochrome c release (Desagher, S., A. Osen Sand, A. Nichols, R. Eskes, S. Montessuit, S. Lauper, K. Maundrell, B. Antonsson, and J.C. Martinou, 1999. Bid-induced conformational change of Bax is responsible for mitochondrial cytochrome c release during apoptosis. *J*. *Cell Biol.* 144:891-901; Eskes, R., S. Desagher, B. Antonsson, and J.C. Martinou, 2000. Bid induces the oligomerization and insertion of Bax into the outer mitochondrial membrane. *Mol. Cell. Biol.* 20:929-935). Secondly, caspase-9 can activate procaspase-8 via the caspase-3 and -6 cascade, thus amplifying the receptor-derived signal and via Bid cleavage also the mitochondrial pathway (Wesselborg, S., I.H. Engels, E. Rossmann, A. Stepczynska, M. Los, and K. Schulze-Osthoff, 1999. Anticancer drugs induce caspase-8/FLICE activation and apoptosis in the absence of CD95 receptor/ligand interaction. *Blood* 93:3053-3063; Los, M., S. Wesselborg, and K. Schulze-Osthoff, 1999. The role of caspases in development, immunity, and apoptotic signal transduction: lessons from knockout mice. *Immunity* 10:629-639).

A crucial step controlling the apoptosome pathway is the release of cytochrome c from mitochondria into the cytosol. It is a rapid and irreversible process that appears to be both energy- and caspase-independent (Goldstein, J.C., N.J. Waterhouse, P. Juin, G.I. Evan, and D.R. Green, 2000. The coordinate release of cytochrome c during apoptosis is rapid, complete and kinetically invariant. *Nat. Cell Biol.* 2:156-162). The mechanism by which cytochrome c translocates to the cytosol during apoptosis has not been elucidated in detail and is still a matter of debate. Much of the controversy has focussed on the mode of action of the pro-apoptotic Bcl-2 family members, such as Bad, Bak, Bax and Bid, which may facilitate the release of cytochrome c, whereas the anti-apoptotic proteins Bcl-2 and Bcl-x_{L} suppress the translocation of cytochrome c (Pellegrini M., A. Strasser, 1999. A portrait of the Bcl-2 protein family: life, death, and the whole picture. *J. Clin. Immunol.* 19:365-377; Gross A., J.M. McDonnell, S.J. Korsmeyer, 1999. Bcl-2 family members and the mitochondria in apoptosis. *Genes Dev.* 13:1899-1911). The functions of the pro-apoptotic Bcl-2 members have been proposed to involve the formation of pores in the outer mitochondrial membrane through which cytochrome c diffuses. Other models suggest that these proteins affect channels in the outer and the inner mitochondrial membrane, such as the permeability transition pore, thereby inducing hyperpolarization and permeability transition (Marchetti, P., M. Castedo, S.A. Susin, N. Zamzami, T. Hirsch, A. Macho, A. Haefner, F. Hirsch, M. Geuskens, and G. Kroemer, 1996. Mitochondrial permeability transition is a central coordinating event of apoptosis. *J. Exp. Med.* 184:1155-1160). It has been proposed that these events may cause the entry of water and solutes, matrix swelling and rupture of the outer membrane, which allows the passive release of cytochrome c. However, it has been observed that in many cell types the release of cytochrome c occurs before or even in the absence of a change in mitochondrial permeability (Goldstein, J.C., N.J. Waterhouse, P. Juin, G.I. Evan, and D.R. Green, 2000. The coordinate release of cytochrome c during apoptosis is rapid, complete and kinetically invariant. *Nat. Cell Biol.* 2:156-162), suggesting that this process involves additional or other mechanisms than opening of the permeability transition pore. Suppression of the anti-apoptotic members of the Bcl-2 family or activation of the pro-apoptotic members therefore leads to an alternation of the mitochondrial membrane permeability resulting in release of cytochrome c into the cytosol.

The deregulation of apoptosis underlies the pathogenesis of a variety of diseases. Disorders associated with decreased apoptosis include cancer (e.g. follicular lymphomas, carcinomas with p53 mutations, hormone-dependent tumors), autoimmune disorders (e.g. systemic lupus erythematosus, rheumatoid arthritis, psoriasis, inflammatory bowel disease, autoimmune diabetes mellitus), and viral infections (e.g. herpesvirus-, poxvirus-, adenovirus-infections). Disorders associated with increased apoptosis are supposed to include AIDS, neurodegenerative disorders (e.g. Alzheimer's disease, Parkinson's disease, amyotrophic lateral sclerosis, retinitis pigmentosa, spinal muscular atrophy, cerebellar degeneration), myelodysplastic syndromes (e.g. aplastic anemia), ischemic injury (e.g. myocardial infarction, stroke, reperfusion injury), and toxin-induced liver disease through alcohol abuse (Thompson, C. B., 1995. Apoptosis in the pathogenesis and treatment of disease. *Science* 267:1456-1462).

A variety of methods has been developed to detect apoptosis through the determination of apoptosis-specific parameters. These methods have been based for example on the detection of phosphatidyl serine (PS) at the cell surface by annexin V binding, DNA-laddering, hypodiploid nuclei, the detection of cytosolic cytochrome c, caspase processing or caspase activity in cells or cell extracts. A number of screening assays for identifying apoptosis-modulating substances has been developed which are based on monitoring of apoptosis-specific events in cells and in cell-free systems.

Los *et al.* (Los M., I. Herr, C. Friesen, S. Fulda, K. Schulze-Osthoff, and K.M. Debatin, 1997. Cross-resistance of CD95- and drug-induced apoptosis as a consequence of deficient activation of caspases (ICE/Ced-3 proteases). *Blood,* 90:3118-3129) disclose the following methods to detect apoptosis: (a) caspase-3 (CPP32) activation is assayed by Western blot through visualising active subunits of this enzyme; (b) caspase-3 activity is shown by Western blot visualisation of cleavage of its specific substrate PARP; and (c) changes in the nucleus and in cell membrane permeability is measured by flow cytometry through a combined staining with DNA dyes propidium iodide and Hoechst 33342. Another group describes a few more methods. DEVD-ase activity of activated caspase-3 can be measured fluorometrically in cell extracts using the substrate z-DEVD-afc. Another parameter, the release of cytochrome c from mitochondria into cytoplasm can be visualized by Western blot of cytoplasmic cell fractions (both described in: Ghibelli L., S. Coppola, C. Fanelli, G. Rotilio, P. Civitareale, A.I. Scovassi, and M.R. Ciriolo, 1999. Gluthathione depletion causes cytochrome c release even in the absence of cell commitment to apoptosis. *FASEB J.* 13:2031-2036).

Similar methods have been described in several publications and patent applications (Rossé T., R. Olivier, L. Monney, M. Rager, S. Conus, I. Fellay, B. Jansen, and C. Borner, 1998. Bcl-2 prolongs cell death survival after Bax-induced release of cytochrome c. *Nature* 391:496-499; Li, F., A. Srinivasan, Y. Wang, R.C. Armstrong, K.J. Tomaselli, and L.C. Fritz, 1997. Cell-specific induction of apoptosis by microinjection of cytochrome c. J. *Biol. Chem.* 272: 30299-30305; Luo, X., I. Budihardjo, H. Zou, C. Slaughter, and X. Wang, 1998. Bid, a Bcl2 interacting protein, mediates cytochrome c release from mitochondria in response to activation of cell surface death receptors. *Cell* 94: 481-490; Erhardt, P., E.J. Schremser, and G.M. Cooper, 1999. B-Raf inhibits programmed cell death downstream of cytochrome c release from mitochondria by activating the MEK/Erk pathway. *Mol. Cell. Biol.* 19: 5308-5315; Kennedy S.G., E.S. Kandel, T.K. Cross, and N. Hay, 1999. Akt/protein kinase B inhibits cell death by preventing the release of cytochrome c from mitochondria. *Mol. Cell. Biol.* 19: 5800-5810; WO 98/58541 and WO 98/02579).

WO 98/58541 is based on the assumption that anti-apoptotic Bcl-2 family members may inhibit apoptosis by binding to cytochrome c. It provides a description of a cell-free screening assay based on the modulation of the interaction between cytochrome c and Bcl-2 family members, preferably between cytochrome c and Bd-2 or Bcl-x_{L} or peptides derived from the Bcl-2 family members. Cytochrome c or Bcl-2 family members may be used in the assay bound to a solid support. The patent covers also antibodies, peptides and peptidomimetics that may modulate apoptosis by influencing interaction between cytochrome c and Bcl-2 family members.

WO 98/02579 further describes a screening assay for the identification of regulators of apoptosis in a cell-free system. The backbone of the assay is a 100 000 *g* fraction of Hela cells (Hela S-100). The readout is e.g. the activation of caspase-3 (CPP32), which can be determined by the detection of the proteolytically activated form of caspase-3 or by the detection of cleaved caspase substrates like PARP or SREBP-2. The detection may be performed by SDS-polyacrylamide gel electrophoresis and autoradiography of radioactively labelled caspase-3 or its substrates, or by Western blot. For test purpose the activation of caspase-3 is initiated in Hela S-100 by addition of nucleotides (preferably dATP) and cytochrome c. The results are compared with those of reference samples. The readout of the apoptotic activity could also be DNA degradation of hamster liver nuclei introduced to the S-100, to which challenge compounds are added. The DNA degradation is assessed by agarose gel electrophoresis. Further, the readout of the apoptotic activity could be the increase of cytoplasmatic cytochrome c.

WO 98/55615 relates to a screening assay for identifying therapeutically active molecules that modulate apoptosis. Cell-free assays designed to test compounds able to compete with cytochrome c for specific binding to Apaf-1 are disclosed. In further cell-free assays apoptosis-modulating substances are identified by measuring the proteolytic cleavage of caspase-3 precursor in the presence or absence of apoptosis-modulating compounds.

WO 99/18856 relates to fluorescent dyes, novel fluorogenic and fluorescent reporter molecules and new assay procedures that can be used to detect the activity of caspases and other enzymes involved in apoptosis in whole cells, cell lines and tissue samples derived from any living organism or organ. The monitoring of apoptosis-specific proteolytic activity can be used for drug screening procedures to identify substances which act as inhibitors or inducers of enzymes of the apoptotic cascade, in particular intracellular caspases. When the enzymes are activated upon induction of apoptosis the fluorogenic or fluorescent reporter molecules are cleaved and respond with a significant increase in fluorescence emission. To detect whether a test substance has an effect on an enzyme involved in the apoptotic cascade, cells are contacted with a test substance and the reporter compound whereby the reporter compound is taken up into the cells, and the test substance either interacts with an external membrane receptor or is taken up into the cells. The change in fluorescence (e.g. magnitude, wavelength) within the cells is recorded and compared to control cells which have only been contacted with the reporter compound and not with the test substance. A change in fluorescence within the test cells in comparison to the control cells is taken as an indication that the test substance has an effect on the apoptotic enzymes.

The above-identified methods to detect apoptosis show a variety of disadvantages, such as being very time-consuming due to complicated experimental procedures and requiring a large number of specific antibodies, or reagents capable of entering the cell. Furthermore, the majority of the methods relies on cell lysis. Some, like e.g. annexin V staining may not be apoptosis-specific, others require large quantities of cells. Most methods usually detect cells in later stages of apoptosis, which are not accessible *in vivo* since at this point apoptotic cells (apoptotic bodies) have already been taken up by phagocytes. The detection of apoptosis by immunostaining has the disadvantage of requiring fixation of the cells using toxic formaldehyde changing the properties of the cell and the cell surface.

The cell-free assays of the prior art detecting cytochrome c and other apoptosis-specific markers cannot exactly predict the ability of apoptosis-modulating substances to penetrate the cellular membrane. An apoptosis-modulating compound should be able to penetrate the intact cell membrane. Additionally, it has been suggested that a number of cellular receptors, proteins, cell constituents and cofactors can influence apoptosis in living cells. The majority of the assays described in the prior art do not take into account these cellular receptors and other cofactors. This way, the risk of false-positive or false-negative identification of apoptosis-modulating substances increases, because it might be possible that a compound identified in these assays might not work in living cells. An apoptosis-modulating substance influencing apoptosis indirectly through one of these cellular receptors or other cofactors would be missed in these assays. Different cell types are supposed to have different receptors and cofactors influencing apoptosis. Therefore, using the cell-free assays, it is impossible to identify cell-type or organo-specific modulators of apoptosis. The fluorescent assay described in WO 99/18856 shows the disadvantage of requiring very specific fluorescent reagents. In addition, it has been described that some caspases can be activated in absence of apoptosis. Therefore, the above-identified assays are not optimal for the identification of apoptosis-modulating substances.

A variety of kits to detect apoptosis are commercially available. These kits use several points in the apoptotic cascade as a target. The kits are designed to detect apoptotis-related events including mitochondrial changes, membrane changes, activation of caspases, DNA-fragmentation, gluthathione level changes and cytosolic cytochrome c. The known uses of the above-identified kits to detect apoptosis have the disadvantages of being very time-consuming due to complicate experimental procedures and requiring a large number of expensive reagents. Furthermore, the described uses usually require the destruction of the cells or cell fractionation experiments.

US-A-5,928,860 discloses a novel process for determining the tolerance, especially the toxicity of gaseous, liquid and/or viscous substances for the human or animal organism. A tissue sample, obtained without proteolytic disintegration, is placed in a chamber which has at least two compartments which are separated from each other by the tissue sample. A nutrient solution is supplied to both sides of the tissue and the tissue is exposed to a test substance on at least one side. The tissue is evaluated to determine the toxicity of the substance.

WO-A-00/17648 discloses antibodies that specifically recognize the new amino terminus of a protein cleaved by a protease during apoptosis. Methods of using and making the antibodies are also provided. The antibodies are particularly useful in methods of detecting apoptosis and testing candidate compounds for enhancing or inhibiting apoptosis.

US-A-6,048,703 discloses methods for the biochemical and immunohistochemical detection of cell apoptosis. The methods utilize the detection and measurement of polypeptide fragments generated during apoptosis. Conditions associated with apoptosis may be detected by the methods of this invention. Methods are also presented for the screening of potential therapeutic compounds which inhibit or stimulate apoptosis. Kits for detection of apoptosis and diagnosis of diseases are also provided.

US-A-5,972,622 describes antibodies or fragments thereof that can be used as indicators of apoptosis. More specifically, this invention relates to antibodies and fragments thereof that selectively bind GP46, a protein whose levels increase significantly upon induction of apoptosis. This invention also relates to the hybridomas that produce anti-GP46 monoclonal antibodies. This invention also discloses a method of detecting cell death by apoptosis in vitro or in vivo by detecting and quantifying GP46 present in biological samples, comprising contacting the sample with the antibodies or fragments to form GP46 immunocomplexes, which may then be detected by the use of known methods. This detection method is useful for research into apoptosis and research relating to diseases in which apoptosis is involved. This method could also be used to diagnose the extent of damage caused by a particular disease or to evaluate the efficacy of drug treatments. The present invention also relates to a method of using the anti-GP46 antibodies or fragments in nuclear medical imaging. The present invention further relates to therapeutic uses of the anti-GP46 antibodies or fragments. The antibodies or fragments can also be incorporated into kits for the detection of apoptosis.

Bleschke, H. et al. discloses in "Deutsche Gesundheitswesen, Vol. 37, No. 5, 1982, pages 204-208 that the bad prognosis of poisonings by Amanita phalloides demands a quick elucidation by: identification of A. phalloides by the patient with the help of figures or toadstools from the stand; macroscopic investigation of cleaning scraps and food leftovers; spore analysis from asservations, contents of the stomach and stool specimens; and exclusion of group's poisonings. A report was given of 12 patients after eating A. phalloides, 1 of whom died. Nine fell ill with typical symptoms. These 9 patients were hemodialyzed relatively soon, before manifestation of the breakdown of the kidneys and/or the liver. In 6 patients, the hemodialysis was started 17-34 h after taking the toadstools. Within this term an effective elimination of the toxin by hemodialysis was possible. The immediate application of the orthograde high enema and the reduction of the enterohepatic circulation by repeated enemas and adsorbents could possibly remove toxins from the gastrointestinal tract. Another form of elimination of toxin was the combined early use of hemodialysis and hemoperfusion. Thioctic acid, G-penicillin and cytochrome c should be given as medicaments.

US-A-4,617,187 describes that cutaneous administration of ubidecarenone, for example, application of ubidecarenone in the form of an ointment has been proven to be effective for treatment of various skin disorders caused by radiation, such as radiation ulcers and radiation dermatitis.

US-A-3,642,978 discloses a process for producing stable cytochrome c preparation, by gelating an aqueous solution containing cytochrome c and gelatin and drying the resulting substance by removing the water while retaining the cytochrome c in a colloidal gel state.

With regard to the above-identified disadvantages of the prior art it would be desirable to have an improved method for the detection of apoptosis and the identification of apoptosis-modulating substances. These methods should be able to detect apoptosis in an earlier stage of the apoptotic process, be easier handled in comparison to prior art methods and should not require destruction of cells.

It is therefore an object of the present invention to provide an improved method for the detection of apoptosis. It is a further object to provide an improved method for the identification of apoptosis-modulating substances, an improved use of an apoptosis detection kit and the use of cytochrome c and/or peptides derived thereof as a medicament, in pharmaceutical compositions, or for the preparation of pharmaceutical compositions for the treatment of diseases with inflammatory manifestation.

These objects have been achieved by the method described in claim 1, the method for the identification of apoptosis-modulating substances described in claim 14, and the use of an apoptosis detection kit for determining apoptosis-specific markers as referred to in claim 19. Preferred embodiments of the present invention are indicated in the dependent claims.

The detection of apoptosis by determining the apoptosis-specific marker cytochrome c according to the present invention allows detection of apoptosis with much more simple, efficient, high-speed, high-throughput methodology, effectively detecting early apoptosis without destruction of the cells. Another advantage of the present invention is that the method can be applied to cells in a natural cell environment without changing or only slightly modifying the properties of the cell or cell surface. The methods to identifiy apoptosis-modulating substances can exactly predict the ability of these substances to penetrate the cellular membrane and take into account cellular receptors and other cofactors influencing apoptosis in living cells. Therefore the risk of false-positive or false-negative identification of apoptosis-modulating substances is significantly lower than that of the prior art methods and it is possible to identify cell-type or organo-specific modulators of apoptosis. Additionally, the assays of the present invention do not require the use of specific cell-permeable fluorescent reagents.

The present invention will become better understood and other aspects, advantages and objectives of the present invention will become apparent from the following description taken in close conjunction with the accompanying Figures. These are for illustration only, and thus are not to be considered as limiting the present invention. Changes and modifications within the spirit and scope of the invention will become apparent to those skilled in the art from the description and the Figures, with the scope of the invention being indicated by the claims.

The invention relates to a method of detection of apoptosis by determining the apoptosis-specific marker cytochrome c, characterized in that presence, amount, or activity of the marker cytochrome c released from at least one cell undergoing apoptosis into an extracellular medium through cellular release mechanisms is determined.

In the present invention the term "cellular release mechanisms" includes all active and passive release mechanisms known to the person skilled in the art to be typical release mechanisms of cells. Examples include diffusion through the cell membrane, exocytosis, or the active or passive escape of apoptosis-specific markers through channels, megachannels, or pores. Combinations of these mechanisms are also possible.

Preferably the amount, or activity of cytochrome c as the apoptosis-specific marker is determined. More preferably the amount of cytochrome c is determined. Combinations of the parameters presence, amount, or activity of cytochrome c can also be determined.

For detection of apoptosis presence, amount, or activity of cytochrome c as apoptosis-specific marker is preferably determined as a function of time.

In another preferred embodiment the extracellular medium is a body fluid, in particular urine, inflammatory fluid, serum or liquor, or cell culture medium.

In another preferred embodiment a sample of urine, inflammatory fluid, serum, liquor, or cells for preparing a cell culture sample is taken from humans or animals.

Examples for the animals used in the invention include non-human mammals, in particular rats, mice, rabbits, cows, horses, sheeps, dogs and cats, and non-mammals, in particular birds, reptiles and fish.

The samples of urine, inflammatory fluid, serum, liquor, or cells for preparing a cell culture sample can be taken from humans or animals using any method known to the person skilled in the art to be suitable for taking samples of the above-identified types from humans or animals.

Liquor includes all cerebrospinal fluids known to the person skilled in the art taken from the brain or the spinal cord.

The cell culture medium of the present invention can be any cell culture medium known to the person skilled in the art to be suitable for growing human and animal cells. Preferably, RPMI-1640 cell culture medium, DMEM, MEM or Grace's insect medium is used. Combinations of these culture media can also be applied but preferably only one single medium is used. All media additionally may comprise heat-inactivated serum, in particular fetal calf serum, new-borne calf serum, horse serum, sheep serum, antibiotics and typical medium additives known to the skilled person.

It might be preferred that apoptpsis is established through comparing presence, amount, or activity of cytochrome c' as apoptosis-specific marker in at least one sample analysed with at least one reference.

The number of samples and references is variable and depends on the specific experimental conditions. In case of several samples and references every known statistical analysis method can be applied to the data obtained from the analysis of the samples and/or the references, e.g. averaging.

In the case of examining body fluid the reference is preferably a standard amount or a determined amount of cytochrome c released into a corresponding body fluid of healthy control subjects or control subjects with apoptosis or necrosis through cellular release mechanisms, or in the case of examining cell culture medium the reference is preferably a standard amount or a determined amount of cytochrome c released into a cell culture medium of cells not undergoing cell death, apoptotic or necrotic cells through cellular release mechanisms, preferably cells of the same cell-type as those of the sample analysed.

In the case of examining body fluid the term "standard amount" includes all amounts known to the person skilled in the art to be typical for the corresponding body fluid of healthy control subjects, or control subjects with apoptosis or necrosis. Additionally, in the case of examining cell culture medium the term "standard amount" includes all amounts known to the person skilled in the art to be typical for cell culture media of cells not undergoing cell death, apoptotic or necrotic cells. The standard amounts of the references are preferably those for similar or identical experimental conditions as used for sample analysis. More preferably the experimental conditions are identical.

In the case of examining body fluid the term "determined amount" indicates that the amount of cytochrome c in the reference is determined experimentally in the corresponding body fluid of healthy control subjects, or control subjects with apoptosis or necrosis. In the case of cell culture medium the term "determined amount" indicates that the amount of cytochrome c in the cell culture medium of the reference is determined experimentally in the cell culture medium of cells not undergoing cell death, apoptotic or necrotic cells. The determination of the references can be performed prior to, parallel to, or after the sample to be analysed. The determination preferably takes place under similar or identical experimental conditions as used for sample analysis. More preferably the experimental conditions are identical.

When examining cell culture medium samples the reference can also be determined through adding a defined amount of a cytochrome c standard to another cell culture medium. This cell culture medium can be of any type known to the person skilled in the art to be a suitable cell culture medium. Preferably the cell culture medium of the reference is similar or identical, more preferably identical, to the cell culture medium of the sample.

In one preferred embodiment, apoptosis is established in the case that the amount of cytochrome c in the sample analysed is similar or equal to the reference, wherein the reference is the amount of cytochrome c in the cell culture medium of apoptotic cells or in the corresponding body fluid of control subjects with apoptosis.

In another preferred embodiment of the present invention, apoptosis is established in the case that the amount of cytochrome c in the sample analysed is larger than the reference, wherein the reference is the amount of cytochrome c in the cell culture medium of cells not undergoing cell death or in the corresponding body fluid of healthy control subjects.

In still another preferred embodiment apoptosis is established in the case that the amount of cytochrome c in the sample analysed is larger than the reference, wherein the reference is the amount of cytochrome c in the cell culture medium of necrotic cells or in the corresponding body fluid of control subjects with necrosis.

The detection of apoptosis through the determination of apoptosis-specific markers other than cytochrome c can be performed with similar or identical procedures as described above.

In another preferred embodiment of the present invention the method is used for the diagnosis and/or for therapy control of diseases and/or processes associated with increased apoptosis, including AIDS; neurodegenerative disorders, in particular Alzheimer's disease, Parkinson's disease, amyotrophic lateral sclerosis, retinitis pigmentosa, spinal muscular atrophy, cerebellar degeneration; myelodysplastic syndromes, in particular aplastic anemia; ischemic injury, in particular myocardial infarction, stroke, reperfusion injury; toxin-induced liver disease through alcohol abuse, or abuse of other substances; diseases with an inappropriate level of production or secretion of hormones, in particular hyperthyroidismus; diseases characterized by inappropriate bone metabolism; metabolic diseases; degenerative processes associated with injury or surgery; and degenerative processes due the hormonal cycle in females, including women.

For diagnosis and/or therapy control of diseases and/or processes associated with increased apoptosis presence, amount, or activity of the apoptosis-specific marker cytochrome c is preferably determined as a function of time. Additionally, presence, amount, or activity of the apoptosis-specific-marker cytochrome c can be determined prior to the start of the therapy to be used as reference. Preferably determination of the amount of cytochrome c is used for diagnosis and/or therapy control.

It might be further preferred, that the method is used for therapy control of diseases associated with decreased apoptosis, including malignant and benign hyperproliferative diseases, in particular lymphomas, carcinomas, sarcomas, other tumors, or leukemias; autoimmune disorders, in particular systemic lupus erythematosus, rheumatoid arthritis, psoriasis, inflammatory bowel disease, or autoimmune diabetes mellitus; and viral infections, in particular those of retroviruses, herpesviruses, poxviruses or adenoviruses.

For therapy control of diseases associated with decreased apoptosis presence, amount, or activity of the apoptosis-specific marker cytochrome c is preferably determined as a function of time. Additionally, presence, amount, or activity of the apoptosis-specific-marker cytochrome c can be determined prior to the start of the therapy to be used as reference. Preferably determination of the amount of cytochrome c is used for therapy control.

According to another preferred embodiment of the invention, the method is used for therapy control of malignant and benign hyperproliferative diseases in the course of chemotherapy, radiotherapy, immunotherapy, surgery or any combination of these therapies.

Examples of the methods used for the therapy of malignant and benign hyperproliferative diseases include all types; of chemotherapy, radiotherapy, immunotherapy, surgery or any combination of these therapies, known to a skilled person. In the present invention chemotherapy includes single drug and multidrug therapy, preferably multidrug therapy.

Presence, amount, or activity of the apoptosis-specific marker cytochrome c' can be determined by any method known to the skilled person to be suitable.

Examples for methods to determine the apoptosis-specific marker cytochrome c, include enzyme-linked immunosorbent assays (ELISA), e.g. in combination with chromogeneous, fluorescent, phosphorescent, or chemoluminescent markers (substrates); homogeneous fluorescence assays; szintillation-proximity assays, surface plasmon resonance assays; electrochemoluminescence assays, sandwich-immuno-assays, assays based on binding to beads in flow cytometers, or assays based on lifetime determination of antibody binding.

The determination of the apoptosis-specific marker cytochrome c, can e.g. be accomplished using labelled markers, preferably labelled antibodies or peptides (e.g. antibody fragments) derived thereof. Examples of possible markers include markers with radioactive, fluorescent, phosphorescent or chemoluminescent properties. Preferably the markers show fluorescent properties.

To detect the fluorescence radiation emitted by the fluorescent markers, all known fluorescence detection techniques known to the skilled person can be applied. Examples include methods basing e.g. on near field spectroscopy, photon distribution analysis, in particular FIDA and 2-D-FIDA, FRET, fluorescence lifetime and fluorescence polarization analysis. The analysis of the data is preferably performed using autocorrelation and/or cross-correlation methods.

It might be further preferred to use confocal spectroscopy for the determination of the apoptosis-specific marker cytochrome c, in particular in combination with surface-based and/or bead-based assay systems.

Specifically, the presence and/or amount of cytochrome c can be determined using any method known to the person skilled in the art to be suitable for the determination of cytochrome c, preferably using an immunoassay, more preferably using immunoprecipitation and immunoblot or an enzyme-linked immunosorbent assay (ELISA). Preferably the amount of cytochrome c is determined using the above-identified methods.

In immunoprecipitation and immunoblots the amount of cytochrome c is preferably determined by visual inspection of the intensities, determination of diameters and/or heights or densitometric scanning of the bands in immunoblots. Combinations of these methods can also be applied.

For immunoprecipitation and immunoblot any anti-cytochrome c antibody known to the person skilled in the art to be suitable for cytochrome c can be used. Examples of suitable anti-cytochrome c antibodies for immunoprecipitation and immunoblot include murine mAb 6H2.B4 and mAb 7H8.2C12. Preferably anti-cytochrome c mAb 6H2.B4 is used in the present invention for immunoprecipitation and mAb 7H8.2C12 for immunoblot.

Detection of antibodies specifically interacting with cytochrome c can be performed with any suitable secondary antibody, or any other detecting reagent, including radioacitively labelled protein A, protein G and streptavidin-conjugated to horseradish peroxidase. The preferred secondary antibody used for incubation is anti-mouse horseradish peroxidase-conjugated secondary antibody.

Blots can be developed by applying any reagent known to the person skilled in the art to be suitable. Enhanced chemoluminescence reagents (ECR) are preferably used for development.

Additionally, it might be further to use imaging techniques, like e.g. nuclear magnetic resonance or roentgen radiation based imaging techniques, in particular computer tomography, for the determination and/or localisation of the apoptosis-specific marker cytochrome c, and/or their complexes with specific antibodies in humans or animals.

Therefore, a method for detecting sites of apoptosis in humans or animals, might be preferred, comprising the steps of:
- preparing labelled antibodies and/or fragments thereof, wherein said antibodies and/or fragments are labelled with a medically acceptable label and are immunoactive with the apoptosis-specific marker cytochrome c;
- administering a safe and effective amount of said labelled antibodies and/or fragments thereof to said humans or animals, and
- detecting the amount or presence of the immune complexes formed between said antibodies and/or fragments thereof and the apoptosis-specific marker cytochrome c, in said humans or animals, wherein the amount or presence of said immune complexes in said humans or animals is correlated with sites of apoptosis in said humans or animals

The antibody used in the above-identified method using imaging systems is preferably a monoclonal antibody.

The present invention also relates to a method for the identification of apoptosis-modulating substances, wherein the method comprises the following steps: a) providing at least one cell culture sample to be analysed; b) contacting said at least one cell culture sample with one putative apoptosis-modulating substance or mixtures of at least two of such substances to be identified; and c) identifying apoptosis-modulating properties through comparing presence, amount, or activity the apoptosis-specific marker cytochrome c in said at least one sample with at least one reference, characterized in that the apoptosis-specific marker cytochrome c released from said at least one cell culture sample into the extracellular cell culture medium through cellular release mechanisms is determined.

Preferably the amount, or activity of the apoptosis-specific marker cytochrome c' is determined. More preferably the amount of the apoptosis-specific marker cytochrome c is determined.

Combinations of the parameters presence, amount, or activity of the apoptosis-specific marker cytochrome c' can be determined.

It might be further preferred to determine the presence, or the amount of cytochrome c, or more preferably the amount of cytochrome c.

For the assay to identify apoptosis-modulating substances according to the present invention any cells known to the skilled person to be suitable for preparing cell culture samples can be used. The cells applied for the preparation of the cell culture samples can be derived from single-cell organisms, humans or animals. Preferably the cells used are taken from humans or animals. Examples for the animals used in the invention include non-human mammals, in particular rats, mice, rabbits, cows, horses, sheeps, dogs and cats, and non-mammals, in particular birds, reptiles and fish.

One putative apoptosis-modulating substance or mixtures of at least two of such substances can be contacted with the cell culture samples. Preferably one single substance with putative apoptosis-modulating properties is used. In the case that apoptosis-modulating properties are established for a mixture of putative apoptosis-modulating substances, the substances of this mixture are additionally re-examined individually one by one.

The putative apoptosis-modulating substances can be contacted with the cell culture samples in any form known to the skilled person to be suitable for contacting cell culture samples with test substances. Substances are preferably added as solid substances, solutions of solid substances, or fluids. Combinations thereof can also be applied.

In the case that the amount of cytochrome c is determined it might be preferred that the reference used in the method for the identification of apoptosis-modulating substances is the amount of cytochrome c released into the cell culture medium of a reference sample through cellular release mechanisms, wherein the reference sample is a cell culture sample of the same cell-type as the sample to be analysed.

In another preferred embodiment apoptosis-enhancing properties of one substance or mixtures of at least two of such substances are established in the case that the amount of cytochrome c released from the cell culture sample analysed is larger than the reference, wherein the reference is the amount of cytochrome c released into the cell culture medium of a reference sample obtained from a cell culture sample in the absence of the apoptosis-modulating substances.

The determination of the references can be performed prior to, parallel to, or after the sample analysis. References are preferably determined under similar or identical experimental conditions as used for sample analysis. More preferably the experimental conditions are identical.

In still another preferred embodiment, apoptosis-inhibiting properties of one substance or mixtures of at least two of such substances are established in the case that the amount of cytochrome c released from the cell culture sample analysed is smaller than the reference, wherein: i) the sample analysed is containing one known apoptosis-enhancing substance or mixtures of at least two of such substances additionally to the apoptosis-modulating substances to be identified, and ii) wherein the reference is the amount of cytochrome c released into the cell culture medium of a reference sample obtained from a cell culture sample containing the same apoptosis-enhancing substances as the sample analysed but in the absence of the apoptosis-modulating substances.

For identifying apoptosis-inhibiting properties, the determination of the references can be performed prior to, parallel to, or after the sample analysis. The references are preferably determined under similar or identical experimental conditions as used for sample analysis. More preferably the experimental conditions are identical. It might be especially preferred that the time periods between the addition of the known apoptosis-enhancing substance or mixtures of at least two of such substances to the sample to be analysed and the reference sample, and analysis of the samples to be analysed and the references are similar or equal. More preferably the time periods are equal.

It might be further preferred, that no apoptosis-modulating properties of one substance or mixtures of at least two of such substances are established in the case that the amount of cytochrome c released from the cell culture sample analysed is similar or equal to the reference, wherein the reference is the amount of cytochrome c released into the cell culture medium of a reference sample obtained from a cell culture sample in the absence of the apoptosis-modulating substances.

As described above, the determination of the references can be performed prior to, parallel to, or after the sample analysis. References are preferably determined under similar or identical experimental conditions as used for sample analysis. More preferably the experimental conditions are identical.

In another preferred embodiment of the invention the above-identified method for the identification of apoptosis-modulating substances is used for the determination of the sensitivity of cells to known apoptosis-enhancing (death-inducing), or apoptosis-inhibiting agents for optimization of therapy of diseases and/or processes associated with apoptosis. More preferably death-inducing agents are applied in the sensitivity tests.

Especially, in the method for identifying apoptosis-modulating substances according to the present invention, cytochrome c can be determined by any method known to the person skilled in the art. Cytochrome c is preferably determined using an immunoassay, more preferably using immunoprecipitation and immunoblot, or an enzyme-linked immunosorbent assay (ELISA). Details for the immunoprecipitation and immunoblot to determine cytochrome c are given above.

It might be further preferred to use confocal spectroscopy for the determination of the apoptosis-specific marker cytochrome c, in particular in combination with surface-based or bead-based assay systems.

The present invention also relates to a use of an apoptosis detection kit for determining presence, amount, or activity of the apoptosis-specific marker cytochrome c released into an extracellular medium through, cellular release mechanisms in a sample to be analysed, wherein the kit comprises a) reagents for the determination of apoptosis-specific markers; and b) instructions for the determination of presence, amount, or activity of the apoptosis-specific marker cytochrome c'.

Additionally, containers to receive the samples to be analysed or cells for preparing cell culture samples might be included into the kit. The containers may have any form known to the person skilled in the art. Suitable containers to be used in the apoptosis detection kit include for example bottles, vials, and test tubes. The containers may be formed from a large variety of materials known in the art. Examples of materials used for the production of containers include glass or polymer-based material.

The reagents used in the kit may comprise any reagents known to the person skilled in the art to be useful for the determination of presence, amount, or activity of the apoptosis-specific marker cytochrom c'.

The instructions to carry out the method of the present invention can be incorporated into the kit in any tangible form known to the person skilled in the art to be useful for detection kits, in particular in the form of written documents, drawings, CD ROMs or any combination thereof.

Preferably the amount, or activity of the apoptosis-specific marker cytochrome c' is determined with the kit. More preferably the amount of the apoptosis-specific marker cytochrome c is determined.

Combinations of the parameters presence, amount, or activity of the apoptosis-specific marker cytochrome c can be determined.

In still another preferred embodiment a sample to be analysed with the kit is a body fluid, in particular urine, inflammatory fluid, serum or liquor, or cell culture medium. It might be further preferred that the sample of urine, inflammatory fluid, serum, liquor, or cells for preparing a cell culture sample is taken from humans or animals.

The apoptosis-specific marker cytochrome c can be determined by any method known to the skilled person to be suitable to determine presence, amount, or activity of cytochrome c. Details are given above.

Especially, cytochrome c can be determined by any method known to the person skilled in the art to be suitable for the determination of cytochrome c. It might be preferred that cytochrome c is determined using an immunoassay, more preferably using immunoprecipitation and immunoblot or an enzyme-linked immunosorbent assay (ELISA). Details for the immunoprecipitation and immunoblot to determine cytochrome c are given above.

It might be further preferred to use confocal spectroscopy for the determination of the apoptosis-specific marker, cytochrome c', in particular in combination with surface-based or bead-based assay systems.

When determining cytochrome c with the kit, the reagents supplied with the kit preferably comprise antibodies for the immunoprecipitation of cytochrome c, washing buffer, anti-cytochrome c antibody for immunoblotting, secondary antibody for incubating the blots, reagents for enhanced chemoluminescence detection of cytochrome c and other typical reagents known to the person skilled in the art. Also included might be labelled markers, preferably fluorescent markers, such as labelled antibodies or fragments thereof.
Fig. 1 (A) shows the extracellular release of cytochrome c after treatment of Jurkat cells with various apoptotic stimuli in comparison to the amount of cytochrome c detected in the remaining cellular extracts (c) of the treated cells.
Fig. 1 (B) shows that cytochrome c is released from the cells into the cell culture medium as a soluble protein.
Fig. 1 (C) shows the dose-dependency of the extracellular release of cytochrome c after treatment of Jurkat cells with various apoptotic stimuli. The quantity of cytochrome c was determined by densitiometric analysis. The graphs display the amount of cytochrome c in the culture medium relative to the total amount of cytochrome c present in medium and cells.
Fig. 2 (A) shows the time-dependency of the release of extracellular cytochrome c and LDH activity in the supernatant.
Fig. 2 (B) shows the assessment of apoptosis induction and progression through the measurement of hypodiploid DNA-formation by flow cytometry. The percentage of apoptotic hypodiploid cells is shown on the right of the histograms.
Fig. 2 (C) shows the relative kinetics of the extracellular release of cytochrome c and LDH activity.
Fig. 3 (A) shows the cytochrome c release into the extracellular medium of apoptotic cells in comparison to untreated or necrotic cells. Also shown is the detection of cell death by PI (propidium iodide) uptake and of apoptosis by detecting hypodiploid nuclei, both by flow cytometry. The percentage of dead cells or apoptotic hypodiploid cells is shown on the right of the histograms.
Fig. 3 (B) shows the extracellular release of cytochrome c of apoptotic cells in comparison to untreated or necrotic cells using a different necrosis inducing substance than in 3 (A). Also shown is the detection of cell death by PI (propidium iodide) uptake and of apoptosis by detecting hypodiploid nuclei, both by flow cytometry. The percentage of dead cells or apoptotic hypodiploid cells is shown on the right of the histograms.
Fig. 4 shows the cytochrome c release into the serum of a patient with acute myeloid leukemia (AML patient) upon chemotherapy in comparison to the amount of serum-cytochrome c of a healthy control subject (control). As a positive control the amount of cytochrome c in total cell extracts from peripheral blood mononuclear cells (PBMC) of a healthy control is shown.
Fig. 5 shows the time-depenency of the cytochrome c release into the serum of chemotherapy-treated patients (Table 1).

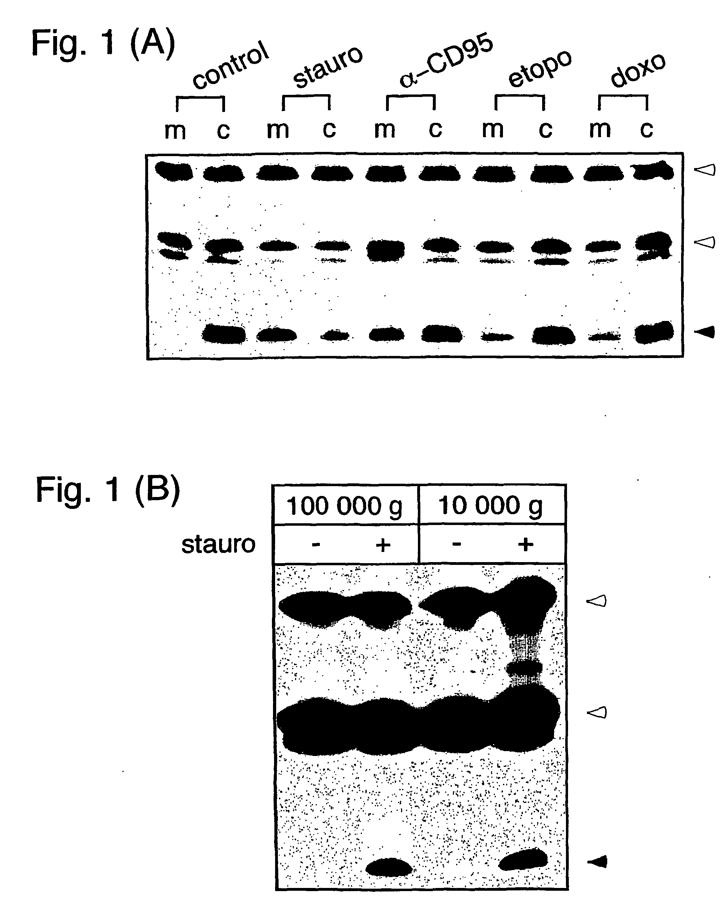

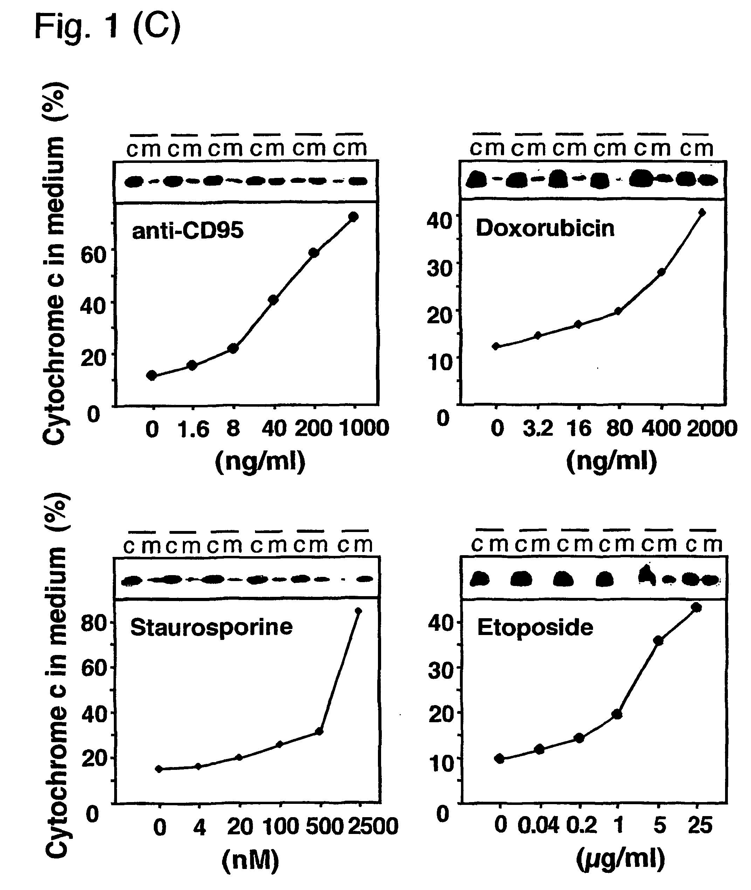

Fig. 1 (A) shows the extracellular release of cytochrome c after treatment of Jurkat cells with various apoptotic stimuli in comparison to the amount of cytochrome c detected in the remaining cellular extracts (c) of the treated cells. 5 x 10⁶ Jurkat cells were cultured in growth medium and either left untreated (control) or stimulated for 16 h with staurosporine (stauro, 2,5 µM), anti-CD95 mAb (α-CD95, 1 µg/ml), etoposide (etopo, 25 µg/ml) or doxorubicin (doxo, 2 µg/ml). Cytochrome c was precipitated from the cell culture medium (m) and the cellular extracts (c) with antibodies against the native molecule. The immunoprecipitated material was separated by SDS-PAGE and analysed by immunoblotting. Open arrowheads indicate heavy chain and light chain of anti-cytochrome c antibody; closed arrowhead indicates cytochrome c. As shown in Fig. 1 (A) large amounts of cytochrome c are found in the cell culture medium of Jurkat T-cells upon apoptosis induction by the protein kinase inhibitor staurosporine, agonistic anti-CD95 antibody and the anticancer drugs etoposide and doxorubicin. For intercomparison, the corresponding cellular fractions are also shown in Fig. 1 (A). It can be seen that the apoptotic event is accompanied by a decrease of cytochrome c in the corresponding cellular fractions.

Fig. 1 (B) shows that cytochrome c is released from the cells into the cell culture medium as a soluble protein. Supernatants of Jurkat cells treated for 16 h with staurosporine (stauro, 2,5 µM) were centrifuged for a period of 30 min at 10 000 *g* or 100 000 *g* and immunoblotted as described above to determine cytochrome c (+). For intercomparison cell culture samples not treated with staurosporine (stauro) (-) are also shown. Cytochrome c was not recovered in the pellets but was found to be still present in the supernatants, indicating that it was not associated with apoptotic bodies or other membrane fractions and was indeed released as a soluble protein.

Fig. 1 (C) shows the dose-dependency of the extracellular release of cytochrome c after treatment of Jurkat cells with various apoptotic stimuli. 5 x 10⁶ Jurkat cells were cultured in growth medium and stimulated for 15 h with the indicated concentrations of anti-CD95 mAb and staurosporine or for 24 h with the indicated concentrations of etoposide or doxorubicin. Cytochrome c was precipitated from the culture medium (m) and the cellular extracts (c) with antibodies against the native molecule. The immunoprecipitated material was separated by SDS-PAGE and analysed by immunoblotting. The quantity of cytochrome c was determined by densitiometric analysis. The graphs display the amount of cytochrome c in the culture medium relative to the total amount of cytochrome c present in medium and cells. The extracellular release of cytochrome c in response to the pro-apoptotic agents is dose-dependent. The amount of cytochrome c released correlates strongly with the induction of apoptosis in Jurkat cells. Similar results (not shown) can be obtained for murine L929 cells.

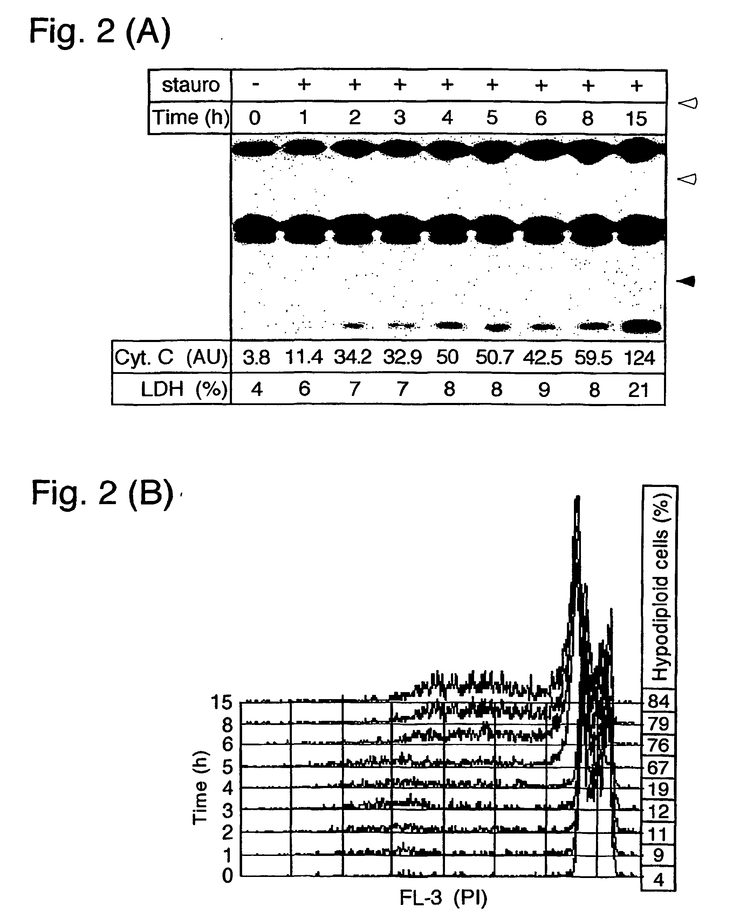

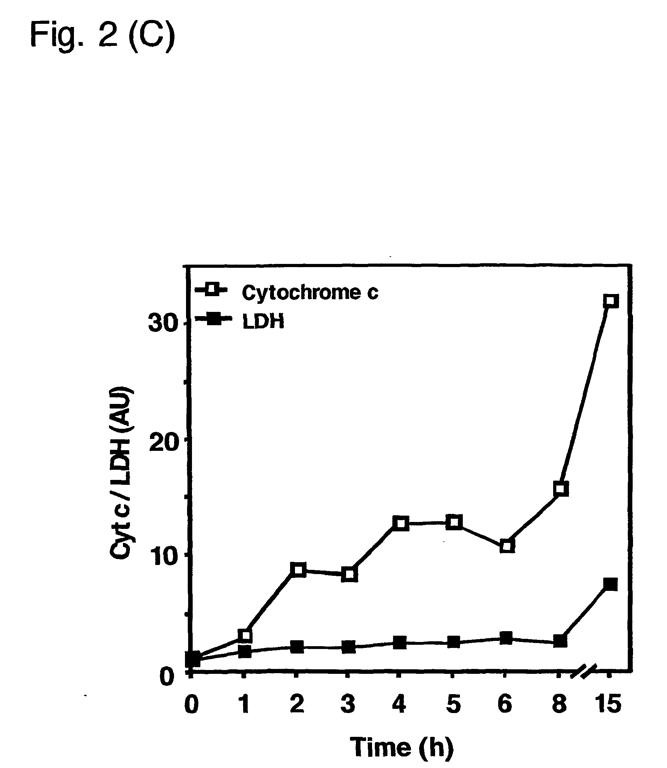

Fig. 2 (A) shows the time-dependence of the release of extracellular cytochrome c and LDH activity in the supernatant. 1 x 10⁶ Jurkat cells were incubated with medium or staurosporine (stauro, 2,5 µM) and harvested after the indicated time points (+). A cell culture sample prior to applying staurosporine and a cell culture sample after 15 h without the addition of staurosporine are shown for intercomparison (-). Release of cytochrome c was determined by immunoprecipitation of the medium fractions and subsequent immunoblotting. Heavy and light chain of anti-cytochrome c antibody and cytochrome c are indicated by open and closed arrowheads, respectively. The LDH activity in the supernatant of apoptotic cells represents the percentage of total LDH activity in the sample. The amounts of cytochrome c in the supernatants indicate the absolute densitometric values. The release of cytochrome c from the cell is a rapid process. Already one hour after apoptosis induction with staurosporine cytochrome c could be detected in the extracellular medium. The parallel measurement of the LDH activity indicated that the release of LDH into supernatants of apoptotic cells occurred much later and was less pronounced than the cytochrome c release. Only about 20 % of the total LDH were detected in the supernatants of apoptotic cells after 15 hours of staurosporine treatment.

Fig. 2 (B) shows the assessment of apoptosis induction and progression through the measurement of hypodiploid DNA-formation by flow cytometry. The percentage of apoptotic hypodiploid cells is shown on the right of the histograms. The standard deviations of the percentage values were not higher than 9 %. 1 x 10⁶ Jurkat cells were incubated with medium or staurosporine (stauro, 2,5 µM) and harvested after the indicated time points. As shown in Fig. 2 (B), formation of hypodiploid nuclei was not observed until 5 h after triggering apoptosis. Therefore, the release of cytochrome c as indicated in Fig. 2 (A) is detectable at a much earlier stage of apoptosis than the formation of hypodiploid cells and the extracellular release of cytochrome c can be regarded as an early and sensitive indicator for apoptosis.

Fig. 2 (C) shows the relative kinetics of the extracellular release of cytochrome c and LDH activity. To visualize the relative quantities of cytochrome c and LDH activity in the culture medium, the absolute densitometric values of cytochrome c and the percentage of released LDH from Fig. 2 (A) were devided by the control values (t = 0 h). A comparison of both events showed that the extracellular release of cytochrome c was stronger and occurred much earlier than the release of LDH.

The ratio of the amount of cytochrome c and the LDH activity in the extracellular medium is useful as therapy control, e.g. during chemotherapy. In a preferred embodiment the determined ratio is used for optimal adjustment of the chemotherapy doses applied to the patients. Chemotherapeutica in very high doses cause necrosis, whereas in therapeutic doses they induce apoptosis.

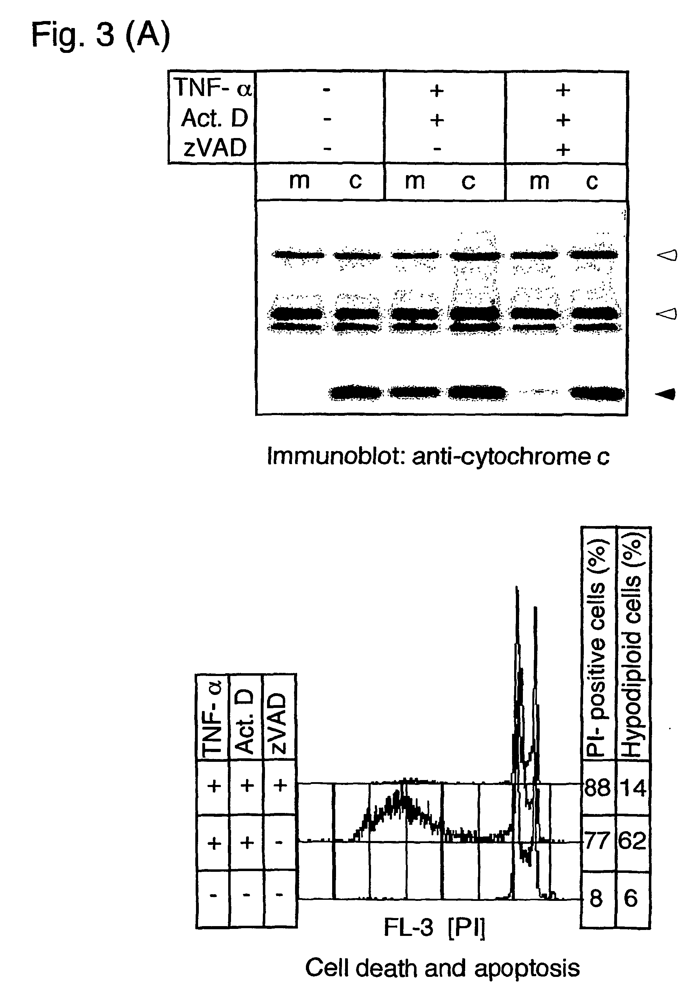

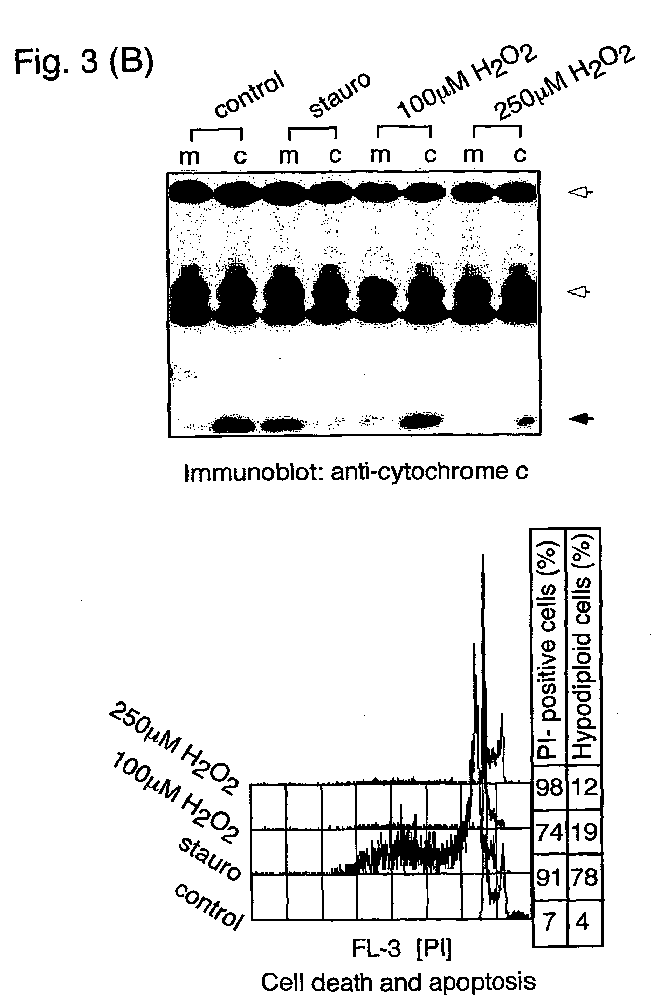

Fig. 3 (A) shows the cytochrome c release into the extracellular medium of apoptotic cells in comparison to untreated or necrotic cells. Also shown is the detection of cell death by PI (propidium iodide) uptake and of apoptosis by detecting hypodiploid nuclei, both by flow cytometry. The percentage of dead cells or apoptotic hypodiploid cells is shown on the right of the histograms. 3 x 10⁶ L929 cells were incubated with medium or with TNF-α (10 ng/ml) plus actinomycin D (Act. D, 1 µg/ml) in the absence or presence of the caspase inhibitor zVAD-fmk (benzyloxycarbonyl-Val-Ala-Asp-fluoromethylketone, zVAD, 100 µM) for 8 h. The release of cytochrome c was determined by immunoprecipitation of culture medium (m) and for intercomparison of corresponding cellular extracts (c) and subsequent immunoblotting. Heavy and light chain of the anti-cytochrome c antibody and cytochrome c are indicated by open and closed arrowheads, respectively. As also shown, cell death was detected by PI (propidium iodide) uptake and apoptosis by detecting hypodiploid nuclei, both by flow cytometry. The standard deviations of the percentage values were not higher than 7%. The stimulation of L929 cells with TNF-α plus actinomycin D induces apoptosis, whereas the same stimuli in presence of the broad-spectrum caspase inhibitor zVAD-fmk induce necrosis (Vercammen, D., R. Beyaert, G. Denecker, V. Goossens, G. Van Loo, W. Declercq, J. Grooten, W. Fiers, and P. Vandenabeele, 1998. Inhibition of caspases increases the sensitivity of L929 cells to necrosis mediated by tumor necrosis factor. *J. Exp. Med.* 187:1477-1485). Concomitant measurement of the DNA content and propidium iodide uptake confirmed these data. In the absence of zVAD-fmk, the cells underwent apoptosis indicated by the formation of hypodiploid nuclei. In contrast, blocking the apoptotic pathway through caspase inhibition by zVAD-fmk resulted in necrosis, as shown by the increased uptake of propidium iodide in the absence of degraded DNA. Extracellular cytochrome c release was only observed upon apoptotic not during necrotic cell death.

Fig. 3 (B) shows the extracellular release of cytochrome c of apoptotic cells in comparison to untreated or necrotic cells using a different necrosis inducing substance than in 3 (A). Also shown is the detection of cell death by PI (propidium iodide) uptake and of apoptosis by detecting hypodiploid nuclei, both by flow cytometry. The percentage of dead cells or apoptotic hypodiploid cells is shown on the right of the histograms. 1 x 10⁶ cells were cultured in normal growth medium (control), or in the presence of staurosporine (stauro, 2,5 µM) or the indicated concentrations of H₂O₂ for 18 h. The release of cytochrome c was determined by immunoprecipitation of culture medium (m) and for intercomparison of corresponding cellular extracts (c) and subsequent immunoblotting. Heavy and light chain of the anti-cytochrome c antibody and cytochrome c are indicated by open and closed arrowheads, respectively. As also shown, cell death was detected by PI (propidium iodide) uptake and apoptosis by detecting hypodiploid nuclei, both by flow cytometry. The standard deviations of the percentage values were not higher than 10%. Treatment of Jurkat T-cells with 100 µM and 250 µM hydrogen peroxide induced necrotic death, as confirmed by PI uptake in the absence of hypodiploid nuclei formation. Similarly to the first approach (Fig. 3 (A)), no cytochrome c release into the extracellular cell culture medium was observed upon necrotic cell death.

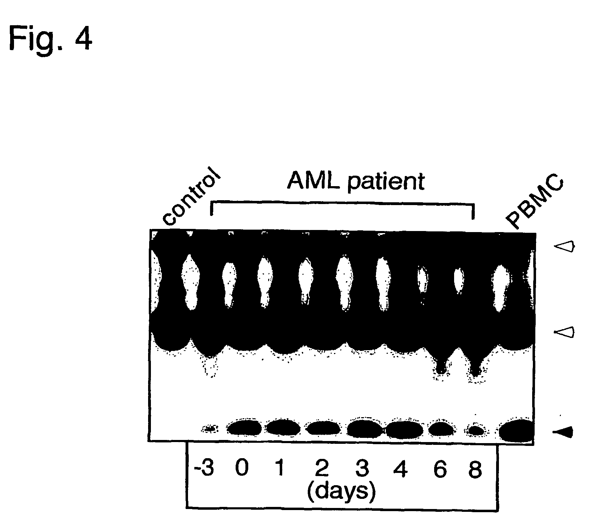

Fig. 4 shows the cytochrome c release into the serum of a patient with acute myeloid leukemia (AML patient) upon chemotherapy in comparison to the amount of serum-cytochrome c of a healthy control subject (control). As a positive control the amount of cytochrome c in total cell extracts from peripheral blood mononuclear cells (PBMC) of a healthy control is shown. Sera (4 ml) obtained from a healthy donor (control) and from a patient with acute myeloid leukemia (AML patient) before and during the chemotherapy were precleared from residual immunoglobulins with protein G. Cytochrome c was immunoprecipitated and subsequently detected by Western blotting. The first day of chemotherapy was considered as day 0; at day 0 the serum was withdrawn after the end of a 12 h infusion with chemotherapeutic drugs. As a positive control total cell extracts from 10⁶ PBMC of a healthy control were used. Heavy and light chain of anti-cytochrome c antibody and cytochrome c are indicated by open and closed arrowheads, respectively. Human sera from six oncologic patients receiving combined chemotherapy were screened. Fig. 4 shows the results of one representative patient. The sera from all six patients with various hematological malignancies and different chemotherapy protocols contained elevated amounts of cytochrome c, when compared to healthy controls. Already a few hours after the onset of chemotherapy with a combination of cytarabine, daunorubicin and thioguanin the amounts in the serum increased dramatically. The chemotherapy reduced the number of white blood cells from 19 800 per µl at day zero to 1200 per µl at day eight, and consequently serum amount of cytochrome c decreased between day six and eight. Comparable results were obtained with serum samples of the other five patients. The slightly elevated basal amount of cytochrome c in the serum of patients as compared to healthy controls may indicate an increased cell turnover in these persons.

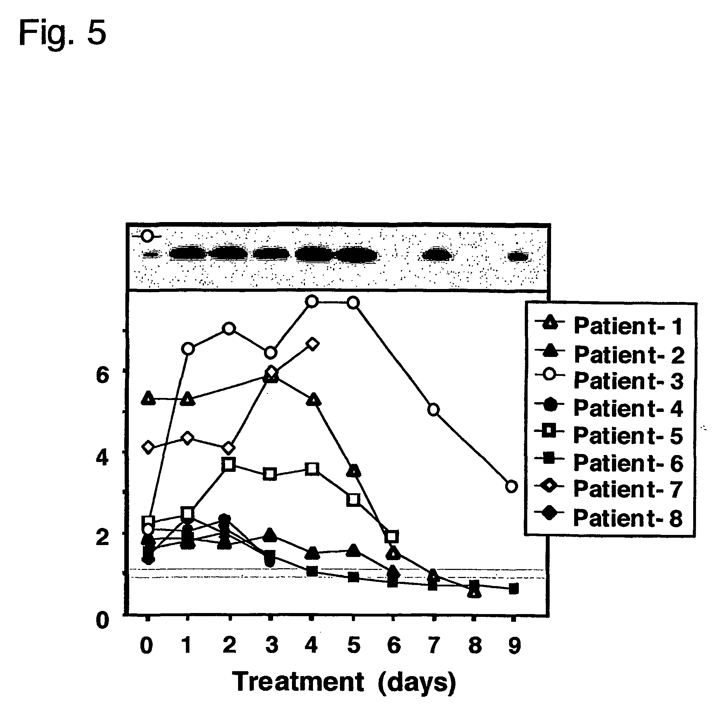

Fig. 5 shows the time-depenence of the cytochrome c release into the serum of chemotherapy-treated patients (Table 1). Sera (3 ml) obtained from different patients as indicated in Table 1 before and during chemotherapy were precleared from residual immunoglobulins with protein G. Cytochrome c was immunoprecipitated and detected by Western blotting. A sample from a healthy control was incorporated into each Western Blot and was used as an internal standard to normalize the data from the different patients. The amount of cytochrome c in the sera samples were quantified by densitometric analysis and calculated as the ratio relative to the value of the control, which was set to 1. Day 0 indicates a sample taken prior to the start of the therapy. The first day of chemotherapy was considered as day 1. At day 1 the serum was withdrawn 8-12 h after the start of the infusion with chemotherapeutic drugs. Healthy control subjects showed only low detectable levels of serum cytochrome c. The gray horizontal bar indicates the range of measured serum cytochrome c in the controls. The top pannel of the graph shows a Western blot of a representive patient. The symbol in the upper left corner of the Western blot indicates the corresponding densitometric analysis. The serum levels of the 8 patients revealed serum cytochrome c increase in the course of chemotherapy, although the kinetic and the extent of increase was variable from patient to patient. The increase of cytochrome c in the serum could be observed already a few hours after the onset of the chemotherapy. The amount of extracellular cytochrome decreased again in the later stages of chemotherapy, reaching levels similar to those of the healthy controls.

For the determination of the relative ratio of the amount of cytochrome c in the sera of the patients and the controls, several other methods can also be applied. E.g. the control can represent the average value of several separate control samples, or a mixture of several control samples, for which one value is determined.

As indicated by the presented data (Figs. 1 to 5) the method of detection of cytochrome c in the extracellular medium according to the present invention is a more simple, more efficient, high-speed, high-throughput methodology for effectively detecting early apoptosis without destruction of the cells.

### Materials and cell culture

The human Jurkat T-cell line and mouse L929 fibroblasts were grown at 37°C, 5% CO₂, in RPMI-1640 medium supplemented with 10% heat-inactivated fetal calf serum, penicillin and streptomycin (all from GIBCO BRL). Etoposide, actinomycin D and doxorubicin were purchased from Sigma and staurosporine from Roche Diagnostics. All stimuli were dissolved in ethanol and kept at-70°C. An agonistic anti-CD95 mAb was obtained from BioCheck and TNF-α from the laboratory of Dr. W. Fiers (Ghent, Belgium). The broad-range caspase inhibitor benzyloxycarbonyl-Val-Ala-Asp-fluoromethylketone (zVAD-fmk) was purchased from Enzyme Systems (Dublin, CA); all other chemicals were from Merck KG or Roth GmbH.

### Serum sample processing

To obtain the data shown in Fig. 4, sera (4 ml per person) from six patients (five males, one female, age 20-48) with hematological malignant diseases were analysed. Three patients suffered from acute myeloid leukemia (AML), two from acute lymphoblastic leukemia (ALL) and one from a relapsed high-grade non-Hodgkin's lymphoma (NHL). All patients received multidrug chemotherapy comprising cytarabine, daunorubicin and thioguanin for the AML patients, and vincristine, daunorubicin and prednisolone or cyclophosphamide, topotecan and cytarabine for the ALL patients. The NHL patient was treated with a combination of fortecortin, carmustine, melphalan, cytarabine and etoposide. Serum samples were collected before and at several time points during therapy. Corresponding control samples were collected from eight age- and sex-matched laboratory personnel. All samples were precleared by centrifugation at 10 000 *g*, 4°C for 15 min. Subsequently, the immunoglobulin content was reduced by two rounds of extraction with 1 ml protein G-sepharose (Pharmacia) for 1 h. Precleared sera were kept at -70°C until use for immunoprecipitation. As a positive control for immunoprecipitation, peripheral blood mononuclear cells (PBMC) from healthy donors were isolated from heparinized fresh blood by gradient centrifugation (Leucoprep™, Nycomed Pharma AS), washed twice in PBS and extracted in lysis buffer as described below.

To obtain the data shown in Fig. 5, sera (3 ml per person) from 8 patients (6 male, 2 female) with mostly hematological malignant disease were analysed. Four patients suffered from acute myeloid leukemia (AML), two from non-Hodgin's lymphoma (NHL), one from Morbus Hodgkin's disease (HB) and one from breast carcinoma. All patients received multidrug chemotherapy as indicated in Table 1. Serum samples were collected before and at several time points during therapy. Corresponding control samples were collected from eight age- and sex-matched laboratory personnel. All samples were precleared by centrifugation at 10 000 *g*, 4°C for 15 min. Subsequently, the immunoglobulin content was reduced by two rounds of extraction with 1 ml protein G-sepharose (Pharmacia) for 1 h. Precleared sera were kept at -70°C until use for immunoprecipitation.

### Cell extracts, immunoprecipitation and immunoblotting

To determine the cellular cytochrome c content, cells were collected by centrifugation, washed in cold PBS and extracted in cold lysis buffer (50 mM Tris, pH 7,4, 150 mM NaCl, 1% Triton-X100 containing 3 µg/ml aprotinin, 3 µg/ml leupeptin, 3 µg/ml pepstatin and 2 mM PMSF). Extracellular fractions and cell lysates were precleared by centrifugation at 10 000 *g*, 4°C for 15 min prior to immunoprecipitation. Supernatants were kept at -70°C until use. Immunoprecipitations were performed in a volume of 4 ml in a rotator at 4°C for 4 h using anti-cytochrome c mAb 6H2.B4 (PharMingen) at a final concentration of 0,5 µg/ml. The cytochrome c-mAb complexes were precipitated for 1 h with 40 µl of a 50% slurry of protein G-sepharose in PBS. Precipitates were harvested by short centrifugation (30 000 *g*, 10 s, 4°C) and washed four times with cold washing buffer (20 mM Hepes, pH 7,4, 150 mM NaCl, 10% glycerol, 0,1% Triton X-100, 1 µg/ml aprotinin, 1 µg/ml leupeptin). Proteins were eluted by boiling the precipitates in SDS-loading buffer containing β-mercaptoethanol, separated under reducing conditions on a 12% SDS-polyacrylamide gel and subsequently transferred to a polyvinylidene difluoride (PVDF) membrane (Amersham Buchler GmbH). Equal loading was confirmed by staining of the proteins with Ponceau S. Subsequently, membranes were blocked for 1 h with 5% non-fat dry milk powder in TBST (TBS, 0,05% Tween-20) and then immunoblotted with anti-cytochrome c mouse mAb 7H8.2C12 (PharMingen) for 2 h. After washing in TBST the blots were incubated with anti-mouse horseradish peroxidase-conjugated secondary antibody for 1 h. Finally, the membranes were washed extensively in TBST and developed using ECL reagents (Amersham Buchler GmbH). The amount of immunoprecipitated cytochrome c was determined by densitometric analysis using the NIH image software (National Institutes of Health).

### Measurement of cell death

For determination of apoptosis, Jurkat and L929 cells were seeded in microtiter plates and treated with the cytotoxic agents for the indicated time periods. Apoptotic, hypodiploid nuclei were measured as described previously (Nicoletti, I., G. Migliorati, M.C. Pagliacci, F. Grignani, and C. Riccardi, 1991. A rapid and simple method for measuring thymocyte apoptosis by propidium iodide staining and flow cytometry. *J*. *Immunol. Methods* 139:271-279; Ferrari, D., A. Stepczynska, M. Los, S. Wesselborg, and K. Schulze-Osthoff, 1998. Differential regulation and ATP requirement for caspase-8 and caspase-3 activation during CD95- and anticancer drug-induced apoptosis. J. *Exp. Med.* 188:979-984). Briefly, apoptotic nuclei were prepared by lysing cells in a hypotonic lysis buffer (1% sodium citrate, 0,1% Triton X-100, 50 µg/ml propidium iodide) and subsequently analysed by flow cytometry. In parallel, cell death as assessed by membrane damage was determined by the uptake of propidium iodide (PI, 2 µg/ml in PBS; Sigma) into non-fixed cells. After 10 min red fluorescence (FL-3) was measured by flow cytometry using a FACScalibur® (Becton Dickinson GmbH) and CeliQuest analysis software (FL-3 [PI]).

Cell viability was also determined by monitoring the release of the cytoplasmic enzyme lactate dehydrogenase (LDH), an indicator for cell death. In order to obtain total LDH activity, cells were lysed with 1 % Triton X-100. The percentage of LDH release represents the fraction of LDH activity found in the supernatant with respect to the overall enzyme activity.

**Table 1 :**

| Summary of patients | | | |
|---|---|---|---|
| Diagnosis | Patient number | Therapy protocol | max. amount of cytochrome c (day) |
| AML | 1,3,6 | TAD^{a} | 3,4,2 |
| | 2 | HAM^{b} | 3 |
| NHL | 4 | CHOEP^{c} | 2 |
| | 5 | ICE^{d} | 2 |
| Morbus Hodgkin | 7 | DHAp^{e} | 4 |
| Breast Cancer | 8 | Epirubicin, paclitaxel^{f} | 2 |
| abreviations AML acute myeloid leukemia NHL non-Hodgkin's lymphoma | | | |

| | | | |
|---|---|---|---|
| TAD (thioguanine, cytosine arabinoside, daunorbucin)^{a} | | | |
| HAM (high dose cytosine arabinoside, mitoxantrone)^{b} | | | |
| CHOEP (cyclophosphamide, doxorubicin, vincristine, etoposide, prednisolone) + anti-CD20^{c} | | | |
| ICE (ifosfamide, carboplatin, etoposide)^{d} | | | |
| DHAP (dexamethasone, high dose cytosine arabinoside, cisplatin)^{e} | | | |

a) Büchner T., Urbanitz D., Hiddemann W., et al. In-tensified induction and consolidation with or with-out maintenance chemotherapy for acute myeloid leukemia (AML): two multicenter studies of the German AML Cooperative Group. J. Clin. Oncol. 1985;3:1583-1589.
b) Hiddemann W., Kreutzmann H., Straif K., et al. High-dose cytosine arabinoside and mitox-antrone: a highly effective regimen in refractory acute myeloid leukemia. Blood. 1987;69:744-749.
c) Köppler H., Pfluger K.H., Eschenbach I., et al. Se-quential versus alternating chemotherapy for high grade non-Hodgkin's lymphomas: a ran- domized multicentre trial. Hematol. Oncol. 1991; 9:217-223.
d) Moskowitz C.H., Bertino J.R., Glassman J.R., et al. Ifosfamide, carboplatin, and etoposide: a highly effective cytoreduction and peripheral-blood progenitor- cell mobilization regimen for transplant-eligible patients with non-Hodgkin's lymphoma. J. Clin. Oncol. 1999;17:3776-3785.
e) Velasquez W.S., Cabanillas F., Salvador P., McLaughlin P., Fridrik M., Tucker S., Jagannath S., Hagemeister F.B., Redman J.R., Swan F., et al. Effective salvage therapy for lymphoma with cis-platin in combination with high-dose Ara-C and dexamethasone (DHAP). Blood. 1988;71:117- 122.
f) Luck H.J., Thomssen C., du Bois A., et al. Phase II study of paclitaxel and epirubicin as first-line therapy in patients with metastatic breast cancer. Semin. Oncol. 1997;24 (Suppl 17):S17-35-S17-39. AQ: 2

## Claims

1. A method of detection of apoptosis by determining apoptosis-specific markers, **characterized in that** presence, amount, or activity of cytochrome c released from at least one cell undergoing apoptosis into an extracellular medium through cellular release mechanisms is determined.

2. The method according to claim 1, **characterized in that** the extracellular medium is a body fluid, in particular urine, inflammatory fluid, serum or liquor, or cell culture medium.

3. The method according to claim 2, **characterized in that** a sample of urine, inflammatory fluid, serum, liquor, or cells for preparing a cell culture sample is taken from humans or animals.

4. The method according to any one of claims 2 or 3, **characterized in that** apoptosis is established through comparing the amount of cytochrome c in at least one sample analysed with at least one reference.

5. The method according to claim 4, **characterized in that** in the case of examining body fluid the reference is a standard amount or a determined amount of cytochrome c released into a corresponding body fluid of healthy control subjects or control subjects with apoptosis or necrosis through cellular release mechanisms, or in the case of examining cell culture medium the reference is a standard amount or a determined amount of cytochrome c released into a cell culture medium of cells not undergoing cell death, apoptotic or necrotic cells through cellular release mechanisms, preferably cells of the same cell-type as those of the sample analysed.

6. The method according to claim 5, **characterized in that** apoptosis is established in the case that the amount of cytochrome c in the sample analysed is similar or equal to the reference, wherein the reference is the amount of cytochrome c in the cell culture medium of apoptotic cells or in the corresponding body fluid of control subjects with apoptosis.

7. The method according to claim 5, **characterized in that** apoptosis is established in the case that the amount of cytochrome c in the sample analysed is larger than the reference, wherein the reference is the amount of cytochrome c in the cell culture medium of cells not undergoing cell death or in the corresponding body fluid of healthy control subjects.

8. The method according to claim 5, **characterized in that** apoptosis is established in the case that the amount of cytochrome c in the sample analysed is larger than the reference, wherein the reference is the amount of cytochrome c in the cell culture medium of necrotic cells or in the corresponding body fluid of control subjects with necrosis.

9. The method according to any one of claims 1-8, **characterized in that** the method is used for the diagnosis and/or for therapy control of diseases and/or processes associated with increased apoptosis, including AIDS; neurodegenerative disorders, in particular Alztieimer's disease, Parkinson's disease, amyotrophic lateral sclerosis, retinitis pigmentosa, spinal muscular atrophy, cerebellar degeneration; myelodysplastic syndromes, in particular aplastic anemia; ischemic injury, in particular myocardial infarction, stroke, reperfusion injury; toxin-induced liver disease through alcohol abuse, or abuse of other substances; diseases with an inappropriate level of production or secretion of hormones, in particular hyperthyroidismus; diseases **characterized by** inappropriate bone metabolism; metabolic diseases; degenerative processes associated with injury or surgery; and degenerative processes due the hormonal cycle in females, including women.

10. The method according to any one of claims 1-8, **characterized in that** the method is used for therapy control of diseases associated with decreased apoptosis including malignant and benign hyperproliferative diseases, in particular lymphomas, carcinomas, sarcomas, other tumors, or leukemias; autoimmune disorders, in particular systemic lupus erythematosus, rheumatoid arfihritis, psoriasis, inflammatory bowel disease, or autoimmune diabetes mellitus; and viral infections, in particular those of retroviruses, herpesviruses, poxviruses, or adenoviruses.

11. The method according to claim 10, **characterized in that** the method is used for therapy control of malignant and benign hyperproliferative diseases in the course of chemotherapy, radiotherapy, immunotherapy, surgery or any combination of these therapies.

12. The method according to any one of claims 1-11, **characterized in that** presence, amount, or activity of cytochrome c is determined as a function of time.

13. The method according to claim 11, **characterized in that** the ratio of the amount of cytochrome c and the LDH activity is determined.

14. A method for the identification of apoptosis-modulating substances, wherein the method comprises the following steps:
a) providing at least one cell culture sample to be analysed;
b) contacting said at least one cell culture sample with one putative apoptosis-modulating substance or mixtures of at least two of such substances to be identified; and
c) identifying apoptosis-modulating properties through comparing presence, amount, or activity of apoptosis-specific markers in said at least one sample with at least one reference,
**characterized in that** cytochrome c released from said at least one cell culture sample into the extracellular cell culture medium through cellular release mechanisms is determined.

15. The method according to claim 14, **characterized in that** the reference is the amount of cytochrome c released into the cell culture medium of a reference sample through cellular release mechanisms, wherein the reference sample is a cell culture sample of the same cell-type as the sample to be analysed.

16. The method according to claim 15, **characterized in that** apoptosis-enhancing properties of one substance or mixtures of at least two of such substances are established in the case that the amount of cytochrome c released from the cell culture sample analysed is larger than the reference, wherein the reference is the amount of cytochrome c released into the cell culture medium of a reference sample obtained from a cell culture sample in the absence of the apoptosis-modulating substances.

17. The method according to claim 15, **characterized in that** apoptosis-inhibiting properties of one substance or mixtures of at least two of such substances are established in the case that the amount of cytochrome c released from the cell culture sample analysed is smaller than the reference, wherein:
a) the sample analysed is containing one known apoptosis-enhancing substance or mixtures of at least two of such substances additionally to the apoptosis- modulating substances to be identified, and wherein
b) the reference is the amount of cytochrome c released into the cell culture medium of a reference sample obtained from a cell culture sample containing the same apoptosis-enhancing substances as the sample analysed but in the absence of the apoptosis-modulating substances.

18. The method according to claim 15, **characterized in that** no apoptosis-modulating properties of one substance or mixtures of at least two of such substances are established in the case that the amount of cytochrome c released from the cell culture sample analysed is similar or equal to the reference, wherein the reference is the amount of cytochrome c released into the cell culture medium of a reference sample obtained from a cell culture sample in the absence of the apoptosis-modulating substances.

19. A use of an apoptosis detection, kit for determining presence, amount, or activity of apoptosis-specific markers released into an extracellular medium through cellular release mechanisms in a sample to be analysed, wherein the kit comprises:
a) reagents for the detection of apoptosis-specific markers; and
b) instructions for the determination of presence, amount, or activity of said apoptosis-specific markers,
**characterized in that** the apoptosis-specific marker determined is cytochrome c.

## Patentansprüche

1. Verfahren zum Nachweis von Apoptose durch Bestimmung von apoptosespezifischen Markern, **dadurch gekennzeichnet, dass** die Anwesenheit, Menge oder Aktivität von Cytochrom c bestimmt wird, das von wenigstens einer Zelle, die Apoptose begeht, durch zelluläre Freisetzungsmechanismen in ein extrazelluläres Medium freigesetzt wird.

2. Verfahren gemäß Anspruch 1, **dadurch gekennzeichnet, dass** das extrazelluläre Medium eine Körperflüssigkeit, insbesondere Urin, Entzündungsflüssigkeit, Serum oder Liquor oder ein Zellkulturmedium ist.

3. Verfahren gemäß Anspruch 2, **dadurch gekennzeichnet, dass** eine Probe von Urin, Entzündungsflüssigkeit, Serum, Liquor oder Zellen zur Herstellung einer Zellkulturprobe von Menschen oder Tieren entnommen werden.

4. Verfahren gemäß einem der Ansprüche 2 oder 3, **dadurch gekennzeichnet, dass** die Apoptose festgestellt wird, indem man die Menge an Cytochrom c in wenigstens einer analysierten Probe mit wenigstens einem Bezugswert vergleicht.

5. Verfahren gemäß Anspruch 4, **dadurch gekennzeichnet, dass** im Falle der Untersuchung einer Körperflüssigkeit der Bezugswert eine Standardmenge oder eine bestimmte Menge an Cytochrom c ist, die durch zelluläre Freisetzungsmechanismen in eine entsprechende Körperflüssigkeit von gesunden Kontrollpersonen oder Kontrollpersonen mit Apoptose oder Nekrose freigesetzt wird, oder im Falle der Untersuchung eines Zellkulturmediums der Bezugswert eine Standardmenge oder eine bestimmte Menge an Cytochrom c ist, die durch zelluläre Freisetzungsmechanismen in ein Zellkulturmedium von Zellen, die keinen Zelltod erleiden, apoptotischen oder nekrotischen Zellen, vorzugsweise Zellen desselben Zelltyps wie die analysierte Probe, freigesetzt wird.

6. Verfahren gemäß Anspruch 5, **dadurch gekennzeichnet, dass** die Apoptose in dem Fall festgestellt wird, dass die Menge an Cytochrom c in der analysierten Probe ähnlich oder gleich dem Bezugswert ist, wobei der Bezugswert die Menge an Cytochrom c im Zellkulturmedium von apoptotischen Zellen oder in der entsprechenden Körperflüssigkeit von Kontrollpersonen mit Apoptose ist.

7. Verfahren gemäß Anspruch 5, **dadurch gekennzeichnet, dass** die Apoptose in dem Fall festgestellt wird, dass die Menge an Cytochrom c in der analysierten Probe größer als der Bezugswert ist, wobei der Bezugswert die Menge an Cytochrom c im Zellkulturmedium von Zellen, die keinen Zelltod erleiden, oder in der entsprechenden Körperflüssigkeit von gesunden Kontrollpersonen ist.

8. Verfahren gemäß Anspruch 5, **dadurch gekennzeichnet, dass** die Apoptose in dem Fall festgestellt wird, dass die Menge an Cytochrom c in der analysierten Probe größer als der Bezugswert ist, wobei der Bezugswert die Menge an Cytochrom c im Zellkulturmedium von nekrotischen Zellen oder in der entsprechenden Körperflüssigkeit von Kontrollpersonen mit Nekrose ist.

9. Verfahren gemäß einem der Ansprüche 1 bis 8, **dadurch gekennzeichnet, dass** das Verfahren für die Diagnose und/oder Therapiekontrolle von Krankheiten und/oder Vorgängen verwendet wird, die mit erhöhter Apoptose verbunden sind, einschließlich AIDS, neurodegenerativen Störungen, insbesondere Alzheimer-Krankheit, Parkinson-Krankheit, amyotrophe Lateralsklerose, Retinitis pigmentosa, spinale Muskelatrophie, Kleinhirndegeneration, myelodysplastische Syndrome, insbesondere aplastische Anämie, ischämische Verletzung, insbesondere Myokardinfarkt, Schlaganfall, Reperfusionsschaden, toxininduzierte Leberkrankheit durch Alkoholmissbrauch oder Missbrauch anderer Substanzen, Krankheiten mit einem ungeeigneten Grad der Produktion oder Sekretion von Hormonen, insbesondere Hyperthyreoidismus, Krankheiten, die durch einen ungeeigneten Knochenmetabolismus **gekennzeichnet** sind, Stoffwechselkrankheiten, degenerative Prozesse, die mit Verletzungen oder Operationen verbunden sind, und degenerative Prozesse aufgrund des Hormoncyclus bei weiblichen Individuen einschließlich Frauen.

10. Verfahren gemäß einem der Ansprüche 1 bis 8, **dadurch gekennzeichnet, dass** das Verfahren für die Therapiekontrolle von Krankheiten verwendet wird, die mit verringerter Apoptose verbunden sind, einschließlich bösartigen und gutartigen hyperproliferativen Krankheiten, insbesondere Lymphomen, Karzinomen, Sarkomen, anderen Tumoren oder Leukämien, Autoimmunstörungen, insbesondere systemischer Lupus erythematodes, rheumatoide Arthritis, Psoriasis, entzündliche Darmerkrankung oder Autoimmun-Diabetes sowie virale Infektionen, insbesondere solche mit Retroviren, Herpesviren, Pockenviren oder Adenoviren.

11. Verfahren gemäß Anspruch 10, **dadurch gekennzeichnet, dass** das Verfahren für die Therapiekontrolle von bösartigen und gutartigen hyperproliferativen Krankheiten im Verlauf der Chemotherapie, Radiotherapie, Immuntherapie, Chirurgie oder einer beliebigen Kombination dieser Therapien verwendet wird.

12. Verfahren gemäß einem der Ansprüche 1 bis 11, **dadurch gekennzeichnet, dass** die Anwesenheit, Menge oder Aktivität von Cytochrom c als Funktion der Zeit bestimmt wird.

13. Verfahren gemäß Anspruch 11, **dadurch gekennzeichnet, dass** das Verhältnis der Menge an Cytochrom c und der LDH-Aktivität bestimmt wird.

14. Verfahren zum Identifizieren von apoptosemodulierenden Substanzen, wobei das Verfahren die folgenden Schritte umfasst:
a) Bereitstellen wenigstens einer zu analysierenden Zellkulturprobe;
b) In-Kontakt-Bringen wenigstens einer Zellkulturprobe mit einer mutmaßlichen apoptosemodulierenden Substanz oder Gemischen von wenigstens zwei solcher Substanzen, die identifiziert werden sollen; und
c) Identifizieren von apoptosemodulierenden Eigenschaften durch Vergleich der Anwesenheit, Menge oder Aktivität von apoptosespezifischen Markern in der wenigstens einen Probe mit wenigstens einem Bezugswert;
**dadurch gekennzeichnet, dass** Cytochrom c bestimmt wird, das von der wenigstens einen Zellkulturprobe durch zelluläre Freisetzungsmechanismen in das extrazelluläre Zellkulturmedium freigesetzt wird.

15. Verfahren gemäß Anspruch 14, **dadurch gekennzeichnet, dass** der Bezugswert die Menge an Cytochrom c ist, die durch zelluläre Freisetzungsmechanismen in das Zellkulturmedium einer Bezugsprobe freigesetzt wird, wobei die Bezugsprobe eine Zellkulturprobe desselben Zelltyps wie die zu analysierende Probe ist.

16. Verfahren gemäß Anspruch 15, **dadurch gekennzeichnet, dass** apoptosesteigernde Eigenschaften einer Substanz oder von Gemischen von wenigstens zwei solcher Substanzen in dem Fall festgestellt werden, dass die Menge an Cytochrom c, die von der analysierten Zellkulturprobe freigesetzt wird, größer ist als der Bezugswert, wobei der Bezugswert die Menge an Cytochrom c ist, die in das Zellkulturmedium einer Bezugsprobe freigesetzt wird, die von einer Zellkulturprobe ohne die apoptosemodulierenden Substanzen erhalten wurde.

17. Verfahren gemäß Anspruch 15, **dadurch gekennzeichnet, dass** apoptosehemmende Eigenschaften einer Substanz oder von Gemischen von wenigstens zwei solcher Substanzen in dem Fall festgestellt werden, dass die Menge an Cytochrom c, die von der analysierten Zellkulturprobe freigesetzt wird, kleiner ist als der Bezugswert, wobei:
a) die analysierte Probe neben den zu identifizierenden apoptosemodulierenden Substanzen eine bekannte apoptosesteigernde Substanz oder Gemische von wenigstens zwei solcher Substanzen enthält und wobei
b) der Bezugswert die Menge an Cytochrom c ist, die in das Zellkulturmedium einer Bezugsprobe freigesetzt wird, die von einer Zellkulturprobe erhalten wurde, die dieselben apoptosesteigernden Substanzen wie die analysierte Probe, aber nicht die apoptosemodulierenden Substanzen enthält.

18. Verfahren gemäß Anspruch 15, **dadurch gekennzeichnet, dass** das Fehlen von apoptosemodulierenden Eigenschaften einer Substanz oder von Gemischen von wenigstens zwei solcher Substanzen in dem Fall festgestellt werden, dass die Menge an Cytochrom c, die von der analysierten Zellkulturprobe freigesetzt wird, ähnlich oder gleich dem Bezugswert ist, wobei der Bezugswert die Menge an Cytochrom c ist, die in das Zellkulturmedium einer Bezugsprobe freigesetzt wird, die von einer Zellkulturprobe ohne die apoptosemodulierenden Substanzen erhalten wurde.

19. Verwendung eines Apoptose-Nachweiskits zur Bestimmung der Anwesenheit, Menge oder Aktivität von apoptosespezifischen Markern, die in einer zu analysierenden Probe durch zelluläre Freisetzungsmechanismen in ein extrazelluläres Medium freigesetzt wird, wobei der Kit Folgendes umfasst:
a) Reagentien zum Nachweis von apoptosespezifischen Markern; und
b) Anweisungen für die Bestimmung der Anwesenheit, Menge oder Aktivität der apoptosespezifischen Marker;
**dadurch gekennzeichnet, dass** es sich bei dem apoptosespezifischen Marker, der bestimmt wird, um Cytochrom c handelt.

## Revendications

1. Un procédé de détection d'apoptose par détermination de marqueurs spécifiques de l'apoptose, **caractérisé en ce qu'**on détermine la présence, la quantité ou l'activité du cytochrome c libéré dans un milieu extracellulaire sous l'action de mécanismes de libération cellulaires par au moins une cellule subissant l'apoptose.

2. Le procédé selon la revendication 1, **caractérisé en ce que** le milieu extracellulaire est un fluide corporel, en particulier l'urine, un liquide inflammatoire, le sérum ou un liquide organique, ou un milieu de culture cellulaire.

3. Le procédé selon la revendication 2, **caractérisé en ce qu'**un échantillon d'urine, de liquide inflammatoire, de sérum, de liquide organique, ou des cellules pour la préparation d'un échantillon de culture cellulaire, sont prélevés à des êtres humains ou des animaux.

4. Le procédé selon l'une quelconque des revendications 2 ou 3, **caractérisé en ce que** l'apoptose est établie en comparant la quantité de cytochrome c dans au moins un échantillon analysé avec au moins une référence.

5. Le procédé selon la revendication 4, **caractérisé en ce que**, dans le cas de l'examen de fluide corporel, la référence est une quantité standard ou une quantité déterminée de cytochrome c libéré sous l'action de mécanismes de libération cellulaires dans un fluide corporel correspondant de sujets témoins sains ou de sujets témoins présentant une apoptose ou une nécrose, ou, dans le cas de l'examen d'un milieu de culture cellulaire, la référence est une quantité standard ou une quantité déterminée de cytochrome c libéré sous l'action de mécanismes de libération cellulaires dans un milieu de culture cellulaire de cellules ne subissant pas de mort cellulaire, de cellules apoptotiques ou nécrotiques, de préférence de cellules du même type cellulaire que celles de l'échantillon analysé.

6. Le procédé selon la revendication 5, **caractérisé en ce que** l'apoptose est établie dans le cas où la quantité de cytochrome c dans l'échantillon analyse est similaire ou égale à la référence, la référence étant la quantité de cytochrome c dans le milieu de culture cellulaire de cellules apoptotiques ou dans le fluide corporel correspondant de sujets témoins présentant une apoptose.

7. Le procédé selon la revendication 5, **caractérisé en ce que** l'apoptose est établie dans le cas où la quantité de cytochrome c dans l'échantillon analysé est plus grande que la référence, la référence étant la quantité de cytochrome c dans le milieu de culture cellulaire de cellules ne subissant pas de mort cellulaire ou dans le fluide corporel correspondant de sujets témoins sains.

8. Le procédé selon la revendication 5, **caractérisé en ce que** l'apoptose est établie dans le cas où la quantité de cytochrome c dans l'échantillon analysé est plus grande que la référence, la référence étant la quantité de cytochrome c dans le milieu de culture cellulaire de cellules nécrotiques ou dans le fluide corporel correspondant de sujets témoins présentant une nécrose.

9. Le procédé selon l'une quelconque des revendications 1 à 8, **caractérisé en ce que** le procédé est utilisé pour le diagnostic et/ou le contrôle thérapeutique de maladies et/ou de processus associés à une apoptose accrue, y compris le SIDA ; des maladies neurodégénératives, en particulier la maladie d'Alzheimer, la maladie de Parkinson, la sclérose latérale amyotrophique, la rétinite pigmentaire, l'amyotrophie spinale, la dégénérescence cérébelleuse ; des syndromes myélodysplasiques, en particulier l'anémie aplasique ; une lésion ischémique, en particulier un infarctus du myocarde, un ictus, une lésion de reperfusion ; une maladie hépatique provoquée par une toxine suite à l'abus d'alcool ou l'abus d'autres substances ; des maladies présentant un taux inapproprié de production ou de sécrétion d'hormones, en particulier l'hyperthyroïdie ; des maladies **caractérisées par** un métabolisme osseux inapproprié ; des maladies métaboliques ; des processus dégénératifs associés à un traumatisme ou une intervention chirurgicale ; et des processus dégénératifs causés par le cycle hormonal chez les femelles, y compris les femmes.

10. Le procédé selon l'une quelconque des revendications 1 à 8, **caractérisé en ce que** le procédé est utilisé pour le contrôle thérapeutique de maladies associées à une apoptose réduite, y compris des maladies hyperprolifératives malignes et bénignes, en particulier les lymphomes, les carcinomes, les sarcomes, d'autres tumeurs, ou les leucémies ; des maladies auto-immunes, en particulier le lupus érythémateux, la polyarthrite rhumatoïde, le psoriasis, les maladies intestinales inflammatoires ou le diabète sucré auto-immun ; et des infections virales, en particulier celles causées par des rétrovirus, herpèsvirus, poxvirus ou adénovirus.

11. Le procédé selon la revendication 10, **caractérisé en ce que** le procédé est utilisé pour le contrôle thérapeutique de maladies hyperprolifératives malignes et bénignes au cours de chimiothérapie, de radiothérapie, d'immunothérapie, de chirurgie ou de toute combinaison de ces thérapies.

12. Le procédé selon l'une quelconque des revendications 1 à 11, **caractérisé en ce que** la présence, la quantité ou l'activité de cytochrome c est déterminée en fonction du temps.

13. Le procédé selon la revendication 11, **caractérisé en ce qu'**on détermine le rapport de la quantité de cytochrome c et de l'activité LDH.

14. Un procédé pour l'identification de substances modulatrices d'apoptose, le procédé comprenant les étapes suivantes :
a)fournir au moins un échantillon de culture cellulaire à analyser ;
b)mettre ledit au moins un échantillon de culture cellulaire en contact avec une substance modulatrice d'apoptose présumée ou des mélanges d'au moins deux de ces substances à identifier ; et
c)identifier les propriétés modulatrices d'apoptose par comparaison de la présence, de la quantité ou de l'activité des marqueurs spécifiques de l'apoptose dans ledit au moins un échantillon avec au moins une référence,
**caractérisé en ce qu'**on détermine le cytochrome c libéré dans le milieu extracellulaire de culture cellulaire sous l'action de mécanismes de libération cellulaires par ledit au moins un échantillon de culture cellulaire.

15. Le procédé selon la revendication 14, **caractérisé en ce que** la référence est la quantité de cytochrome c libéré dans le milieu de culture cellulaire d'un échantillon de référence sous l'action de mécanismes de libération cellulaires, l'échantillon de référence étant un échantillon de culture cellulaire du même type cellulaire que celui de l'échantillon à analyser.

16. Le procédé selon la revendication 15, **caractérisé en ce que** des propriétés stimulatrices d'apoptose d'une substance ou de mélanges d'au moins deux de ces substances sont établies dans le cas où la quantité de cytochrome c libéré par l'échantillon de culture cellulaire analysé est plus grande que la référence, la référence étant la quantité de cytochrome c libéré dans le milieu de culture cellulaire d'un échantillon de référence provenant d'un échantillon de culture cellulaire en l'absence des substances modulatrices d'apoptose.

17. Le procédé selon la revendication 15, **caractérisé en ce que** des propriétés inhibitrices d'apoptose d'une substance ou de mélanges d'au moins deux de ces substances sont établies dans le cas où la quantité de cytochrome c libéré par l'échantillon de culture cellulaire analysé est plus petite que la référence, lorsque :
a)l'échantillon analysé contient une substance stimulatrice d'apoptose connue ou des mélanges d'au moins deux de ces substances, en plus des substances modulatrices d'apoptose à identifier, et lorsque
b)la référence est la quantité de cytochrome c libéré dans le milieu de culture cellulaire d'un échantillon de référence provenant d'un échantillon de culture cellulaire contenant les mêmes substances stimulatrices d'apoptose que l'échantillon analysé, mais en l'absence des substances modulatrices d'apoptose.

18. Procédé selon la revendication 15, **caractérisé en ce qu'**aucune propriété modulatrice d'apoptose d'une substance ou de mélanges d'au moins deux de ces substances n'est établie dans le cas où la quantité de cytochrome c libéré par l'échantillon de culture cellulaire analysé est similaire ou égale à la référence, la référence étant la quantité de cytochrome c libéré dans le milieu de culture cellulaire d'un échantillon de référence provenant d'un échantillon de culture cellulaire en l'absence des substances modulatrices d'apoptose.

19. Une utilisation d'une trousse de détection d'apoptose pour déterminer la présence, la quantité ou l'activité de marqueurs spécifiques de l'apoptose libérés dans un milieu extracellulaire sous l'action de mécanismes de libération cellulaires dans un échantillon analyser, la trousse comprenant :
a)des réactifs pour la détection de marqueurs spécifiques de l'apoptose ; et
b)des instructions pour la détermination de la présence, de la quantité ou de l'activité desdits marqueurs spécifiques de l'apoptose,
**caractérisée en ce que** le marqueur spécifique de l'apoptose déterminé est le cytochrome c.
